(19)

Europäisches Patentamt
European Patent Office
Office européen des brevets

(11) **EP 3 932 436 A1**

(12) **EUROPEAN PATENT APPLICATION**
published in accordance with Art. 153(4) EPC

(43) Date of publication:
**05.01.2022 Bulletin 2022/01**

(21) Application number: **20762978.3**

(22) Date of filing: **26.02.2020**

(51) Int Cl.:
**A61L 24/00** *(2006.01)*      **A61K 35/65** *(2015.01)*
**A61K 9/06** *(2006.01)*        **A61P 17/02** *(2006.01)*

(86) International application number:
**PCT/CN2020/076763**

(87) International publication number:
**WO 2020/173459 (03.09.2020 Gazette 2020/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.02.2019   CN 201910140042
20.06.2019   CN 201910536465
05.07.2019   CN 201910603111**

(71) Applicant: **Stomatological Hospital of
Chongqing Medical
University
Chongqing 401147 (CN)**

(72) Inventors:
• **ZHANG, Ximu**
  **Chongqing 401147 (CN)**
• **JI, Ping**
  **Chongqing 401147 (CN)**
• **SONG, Jinlin**
  **Chongqing 401147 (CN)**

(74) Representative: **Lang, Christian
LangPatent Anwaltskanzlei IP Law Firm
Ingolstädter Straße 5
80807 München (DE)**

(54)     **MEDICAL ADHESIVE, AND MANUFACTURING METHOD THEREFOR AND USE THEREOF**

(57)     A medical adhesive and the preparation method, and application thereof. The medical adhesive is in the form of a gel, which contains a giant salamander skin mucus and an aqueous solution; the ratio parts by weight of the giant salamander skin dried powder and the aqueous solution is 1:1 to 1:6, and the weight content of the giant salamander skin mucus freeze-dried powder in the medical adhesive ranges from 14.2% to 50%. The present invention also provides the preparation method for preparing the aforementioned medical adhesive, and at the same time, the aforementioned medical adhesive is applied for the wound.

Fig. 6

EP 3 932 436 A1

**Description**

**FIELD OF THE INVENTION**

The invention belongs to the field of biological materials, and relates to wound treatment, particularly to a medical adhesive prepared from natural ingredients as raw materials and to the preparation method and use of the medical adhesive.

**BACKGROUND OF THE INVENTION**

Closing a surgical wound is an essential step in surgical treatment where more than 60% of the wound will be stitched using sutures and skin staples. However, the stitches lead to the follow-up medical treatment, which not only increases medical visits of the patient, but also increases the risk of hypertrophic scar, scar contracture, and foreign body infection and the like. By adopting a non-suturing method such as biomedical glue, the operation steps can be simplified, the recovery time is shortened, and the nursing quality of the patient is improved.

For wounds that are difficult to heal, the deep wounds caused by puncture and the wounds in the wet environment that are hard to dress, presently, there is no research result showing that medical adhesive can be used to block the wounded surface, promote tissue regeneration and accelerate healing.

Dense connective tissue refers to tissue consisting of a small amount of matrix and cells and plenty of dense fibers; and the fibers are thick and densely arranged to form the tissues having main functions of supporting and connecting, including dermis, tendons and ligaments, etc. The tendons comprise Achilles tendon connecting the calf muscle with the calcaneus bone, and patellar tendon connecting the patella and the tibia. Ligaments comprise extracapsular ligament located around the articular cavity, and intracapsular ligament located within the articular cavity.

Injury of dense connective tissue is a common orthopedic disease. Many reasons can lead to injury to dense connective tissue including overuse, sprain, disease and general aging, etc. As the life of human life is prolonged and more and more middle-aged people are taking part in sports or vigorous exercise, the incidence rate of tendon injury and ligament injury is increasing year by year.

Tendon injury can be divided into acute and chronic injuries, and the reasons include internal or external factors; either internal factors or combination of internal and external factors can cause tendon injury. Tendon injury mainly occurs in sports and working places, which can often lead to pain, stiffness and tendon strength impairment of affected parts. Achilles tendon injury and patellar tendon injury are the two types of most common motion tendon injuries. Tendon injury or ligament injury is still a serious and unresolved problem in clinic. Surgical methods, such as sutures and the like, are currently used for treating fracture or tearing of tendons or ligaments. Lack of blood supply and the like can lead to concurrent infection and slow healing of tendon or ligament injury. Moreover, there is no surgical procedure for repairing injured tendon or ligament to its normal tissue structure and mechanical strength. At the same time, tendon injury can lead to a long-term unhealthy status, and no matter what nursing method is adopted, the disability caused by the tendon injury can last for several months. Regarding the currently available management means for tendon disorders treatment, only a few clinical studies have been completed. Most of the means are still in the stage of preclinical study, and the results of such treatment means are controversial. In rat Achilles tendonitis model induced by collagenase, extracorporeal shock wave therapy has been demonstrated to promote healing of the tendon by inducing expression of TGF-$\beta$ (transforming growth factor-beta) and IGF-1 (insulin-like growth factor 1). In rat Achilles tendonitis model, a 17 hertz pulse magnetic field and an electromagnetic field can improve the arrangement of collagen fibers. In addition, certain cytokines and growth factors such as TGF-$\beta$ and IGF-1, as well as mesenchymal stem cells (MSCs) for gene therapy and tissue engineering are used for the treatment of tendon injury and exhibit promising prospect. However, the price of the growth factor is expensive, and it is easy to degrade, so large economic burden is brought to the patients.

So far, it is not reported that medical adhesive can be used as the adhesive therapy for dense connective tissue to replace the suture treatment method. Due to the fact that the tendon or ligament tissue is continuously stressed by the motion of the human body, the medical adhesive used for bonding the tendon or ligament tissue has to be highly adhesive, it also needs to exert the ability of resisting the tension. At the same time, based on the consideration of postoperative recovery, the adhesive used must have good biocompatibility, easy to be absorbed by tissue and has promising biological safety.

As for clinical treatment and repair of dense connective tissue wound surface or wound, a medical adhesive which has enough supply, good biocompatibility, high bonding strength and ease of biodegradability is lacking in the prior art. In the current medical adhesive technology, cyanoacrylates and fibrin are widely used in clinical as the alternatives of stitching. Cyanoacrylates have obvious cytotoxicity; in the bonding process, heat is generated by severe oxidation reduction reaction; and the defects such as rigidity and difficulty in degradation are shown after bonding. Therefore, it is difficult to apply to large-area and wounds positioned in tensioned body zone; meanwhile, the heat generation, cytotoxicity and carcinogenicity shown in animal experiments limit the further application of cyanoacrylates.

As for fibrin, fibrin can be used in combination with sutures in cosmetic surgery to reduce the number of stitches and the scar hyperplasia, but because of the slow curing and the poor mechanical strength, it is hard to use fibrin alone. It can be understood that the application of these two medical adhesives is limited and is completely unsuitable for adhesive for dense connective tissue.

Giant salamander (Andrias Davidianus) Blanchard is a large amphibian animal which belongs to amphibia class, urodela order and cryptobranchidae family, and it has a common name "baby fish", which is an animal species under national second-class protection. When the giant salamander encounters external stimulation, the skin of the body can secrete a type of mucus. Presently, there are studies showing the method for preparing adhesive or hemostatic agent by using giant salamander skin mucus freeze-dried powder. Regarding the preparation of an adhesive by using giant salamander skin mucus freeze-dried powder, a Chinese patent (CN104815349B) discloses a method for preparing an adhesive from giant salamander mucus, only preliminarily disclosing that the giant salamander mucus is subjected to gamma ray disinfection and then is used for manufacturing the adhesive as well as the adhesive property thereof, while the giant salamander mucilage produced by the method has no scientific description or test for this materials. Another published Chinese patent application CN106581736A simply discloses a method for manufacturing the giant salamander mucus freeze-dried powder, which lacks scientific evidence to support the effects brought by the method. Another published Chinese patent application CN108421080A discloses a preparation method of giant salamander exudate hydrogel. According to this method, external pressure needs to be applied in the gel-forming preparation process and the solvent system is only water. The product can only be formed in a thin film shape and cause inconvenience for use. Moreover, this publication lacks scientific evidence to support the experimental effects in vivo.

The above facts show that medical adhesive prepared from giant salamander skin mucus still has defects demanding prompt solution.

## SUMMARY OF THE INVENTION

According to above drawback of the prior art, the present invention provides the preparation method and application of a medical adhesive contains a giant salamander skin mucus to solve the technical problems. The medical adhesive of the present invention takes advantage of the strong adhesive properties of the giant salamander skin mucus. After the giant salamander skin mucus is prepared to form a freeze-dried powder which is then to sterilize, and the sterilized freeze-dried powder is provided for healing the wound caused by surgical treatment or other wounds. The applications of the medical adhesive include various tissue of the skin system, skeletal system and muscle system, especially for dense connective tissue to bring significant effect.

The medical adhesive of the present invention has better safety, better biocompatibility, degradability, and regeneration promoting effects, as well as antibacterial and hemostatic effects, and is suitable as a tissue adhesive or dressing for surgical wounds or wounds. The medical adhesive is a material that meets the requirement.

According to above objects, the present invention provides a medical adhesive, which contains a giant salamander skin mucus dried powder and an aqueous solution, the ratio parts by weight of the giant salamander skin mucus dried powder and the aqueous solution is 1:1 to 1:6.

According to the objects of the present invention, the medical adhesive is in gel form.

According to the objects of the present invention, the weight percentage of giant salamander skin mucus dried powder in medical adhesive ranges from 14.2% to 50%.

According to the objects of the present invention, the aqueous solution of the medical adhesive is selected from the group consisting of distilled water, physiological buffer, chlorhexidine, blood, plasma, blood cell preparation, platelet-rich plasma (PRP), platelet-rich fibrin (PRF), or any combination of the above.

According to the objects of the present invention, the aqueous solution is selected from the normal saline, phosphate buffer, Tris buffer, citrate buffer or any combination of the above.

According to the objects of the present invention, the gel medical adhesive has a porous structure.

According to the objects of the present invention, the average diameter of aperture of the porous structure is smaller than 116$\mu$m.

According to the objects of the present invention, the average diameter of aperture of the porous structure is between 6$\mu$m to 37$\mu$m.

According to objects of the present invention, the particle size of the giant salamander skin mucus dried powder contained in the medical adhesive is -20 mesh.

According to the objects of the present invention, the particle size of the giant salamander skin mucus dried powder contained in the medical adhesive is -60 mesh to +300 mesh.

According to the objects of the present invention, the present invention provides an application of the medical adhesive, the medical adhesive is applied for the adhesion, repair, and healing of the wounds.

According to the objects of the present invention, in one preferred embodiment, the wounds include wounds located in the epidermis, dermis and subcutaneous connective tissue.

According to the objects of the present invention, in one preferred embodiment, the wounds include wounds located in skeletal muscles, tendons, ligaments, bones and/or connective tissue around bones.

According to the objects of the present invention, in one preferred embodiment, when the medical adhesive is used, the giant salamander skin mucus dried powder and the aqueous solution are directly applied to the wound surface, thereby the medical adhesive is directly formed on the wound.

According to the objects of the present invention, in one preferred embodiment, the giant salamander skin mucus dried powder and the aqueous solution are preliminarily used in advance to form the medical adhesive, which are then used to apply on the wound or to seal the wound.

According to above objects, the present invention also provides a preparation method of medical adhesive, the step includes: providing a giant salamander skin mucus dried powder; sterilizing the giant salamander skin mucus dried powder; and mixing the sterilized giant salamander skin mucus dried powder and an aqueous solution to carry out a gelation process to form the medical adhesive is in gel form, in which the medical adhesive contains the ratio parts by weight of the giant salamander skin freeze-dried powder and the aqueous solution is 1:1 to 1:6, the weight percentage of giant salamander skin mucus dried powder in medical adhesive ranges from 14.2% to 50%.

According to the objects of the present invention, in one preferred embodiment and in above preparation method of the medical adhesive, the step of providing the giant salamander skin mucus dried powder further includes: obtaining a giant salamander skin mucus from a living giant salamander skin; freezing and drying the giant salamander skin mucus; and grinding and crushing the dried giant salamander skin mucus to form the giant salamander skin mucus dried powder.

According to the objects of the present invention, in one preferred embodiment and in above preparation method of the medical adhesive, the particle size of the giant salamander skin mucus dried powder is -20 mesh.

According to the objects of the present invention, in one preferred embodiment and in above preparation method of the medical adhesive, the particle size of the giant salamander skin mucus dried powder is -60 mesh to +300 mesh.

According to the objects of the present invention, in one preferred embodiment and in above preparation method of the medical adhesive, the step of sterilization of the giant salamander skin mucus freeze-dried powder is achieved by using ethylene oxide.

According to the objects of the present invention, in one preferred embodiment and in above preparation method of the medical adhesive, the aqueous solution is selected from distilled water, physiological buffer, chlorhexidine, blood, plasma, blood cell preparation, platelet-rich plasma, and platelet-rich plasma fibrin or any combination of the above.

According to the objects of the present invention, in one preferred embodiment and in above preparation method, the physiological buffer is selected from the distilled water, physiological buffer, chlorhexidine, blood, plasma, blood cell preparation, platelet-rich plasma, and platelet-rich plasma fibrin, or any combination of the above.

## BRIEF DESCRIPTION OF THE DRAWINGS

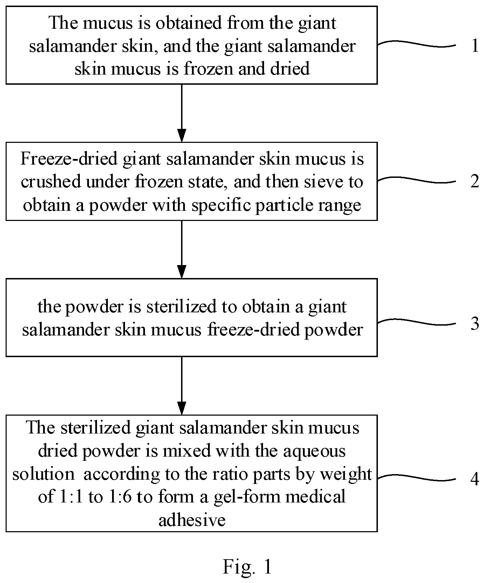

Fig. 1 shows a flow chart of the preparation method of the medical adhesive in accordance with the present invention.

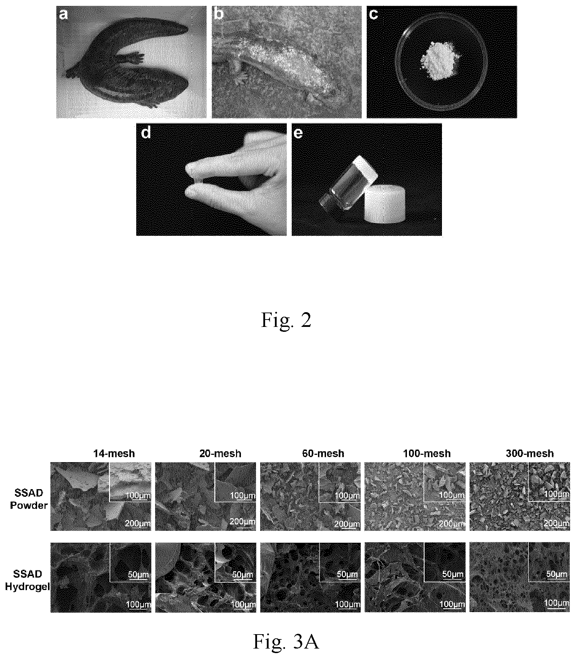

Fig. 2 shows the giant salamander described in embodiment 1 of the present invention (a); mechanical scraping is used to obtain giant salamander skin mucus (b); the giant salamander skin mucus dried powder (c); the types of adhesive drawing of the medical adhesive to the tissue (d); The form of gelatinized of giant salamander skin mucus dried powder (e).

Fig. 3A is the schematic of the scanning electron microscope (SEM) of showing the giant salamander skin mucus dried powder (SSAD powder) with different particle size is obtained by sieving with different meshes, and the giant salamander skin mucus dried powder is hydrated to form a hydrogel state in embodiment 2 of the present invention)image. From this SEM image, the size of the pore size in hydrogel form after hydration is obtained.

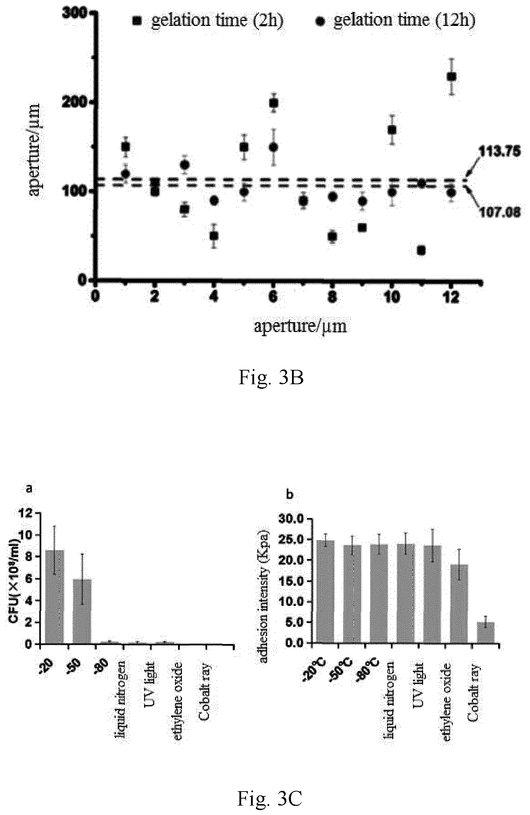

Fig. 3B shows the pore size distribution of the porous structure within the medical adhesive with different hydrogel times (2h and 12h) in embodiment 2 of the present invention.

FIG. 3C shows the sterilization effect data graph (a) and the adhesion intensity data graph (b) achieved by the various sterilization schemes of embodiment 2 of the present invention.

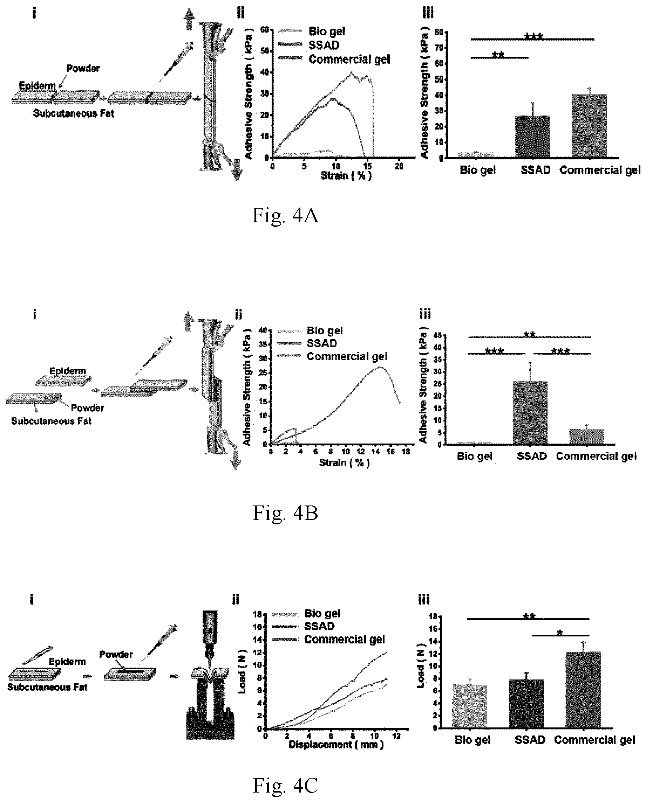

Fig.4A shows the in vitro adhesion performance comparison between the medical adhesive of embodiment 3 of the present invention and two common commercially available medical adhesives, in which pig skin is used as the biological matrix to test the adhesive intensity (n=4) of the medical adhesive for standard wound sealing: the schematic diagram of the improved standard test method for adhesion intensity (ASTM F2458-05) (i); representative strain-stress curve (ii); quantitative comparison of the adhesion strength of different medical adhesives (iii) (statistical difference) : ** $P < 0.01$, *** $P < 0.001$.

Fig. 4B shows the comparison of the in vitro adhesion performance of the medical adhesive of embodiment 3 of the present invention and two common commercially medical adhesives, in which pig skin is used as the biological matrix to test the shear intensity after adhesion of the subcutaneous fat surface of the pig: modified schematic diagram of the standard method of shear test (ASTM F2255-05) (i); representative strain-stress curve (ii); quantitative comparison of the adhesive intensity of different medical adhesives (iii) (statistical difference: ** P<0.01, *** P<0.001).
Fig. 4C shows the comparison of the in vitro adhesion performance of the medical adhesive of embodiment 3 of the present invention and two common commercially available medical adhesives. The pig skin is used as the biological matrix and the schematic diagram (i) of showing modified three-point bend test method is used to determine the elasticity and ductility of pig skin after the medical adhesive is adhered; a representative strain-displacement curve (ii); the quantitative comparison of required load force of different medical adhesives in fixed deformation (11.5%) (iii) (statistical difference: * P<0.05, ** P<0.01, *** P<0.001).

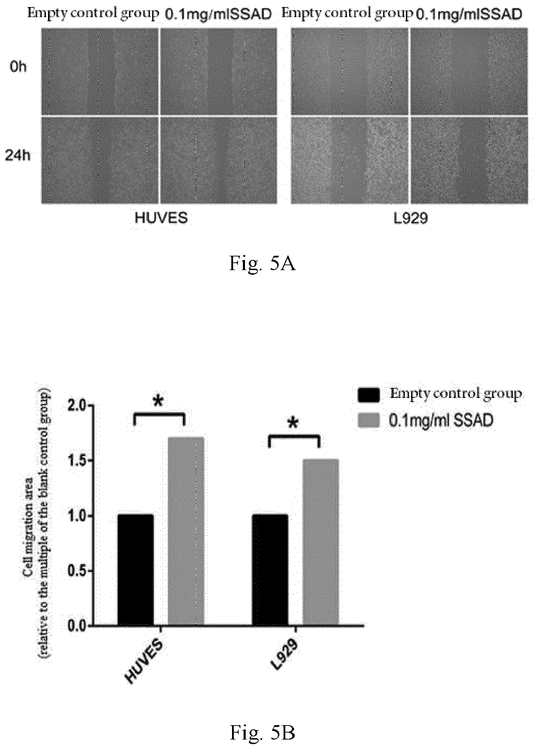

Fig. 5A shows the results of the cell scratch test of the medical adhesive of Experiment 3 of the present invention.
Fig. 5B shows the statistical analysis result of the cell scratch test of the medical adhesive of Experiment 3 of the present invention.

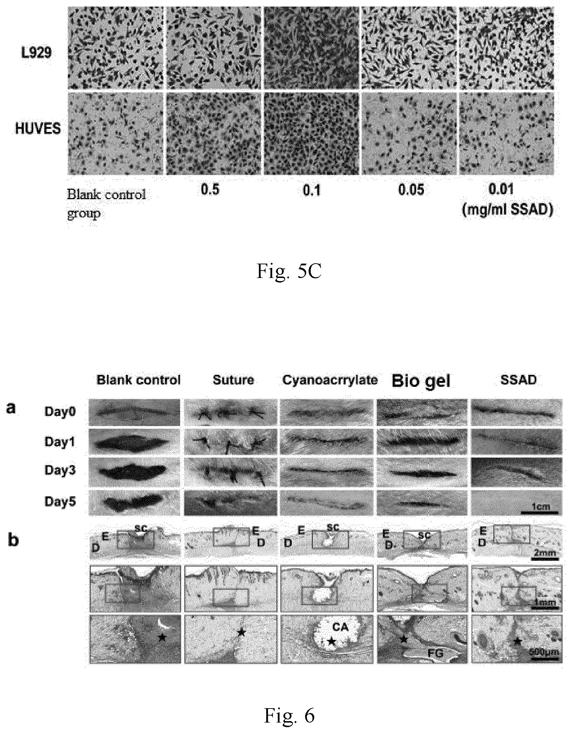

FIG. 5C shows the results of the transwell chamber experiment of the medical adhesive of Experiment 3 of the present invention.
Fig. 6 shows a comparison diagram of in vivo effects of wound sealing methods in the SSAD treatment group and the other 4 groups in embodiment 4 of the present invention, in which Fig. 6a shows the images of rat skin incisions on days 0, 1, 3, and 5; Fig. 6b shows the H&E stained images of the wound tissues, and epidermis (E), dermis (D), and scab (SC), incision position (★), wound area (marked with a frame), undegraded cyanoacrylate adhesive (CA) and fibrin glue (FG) are respectively indicated by labels.

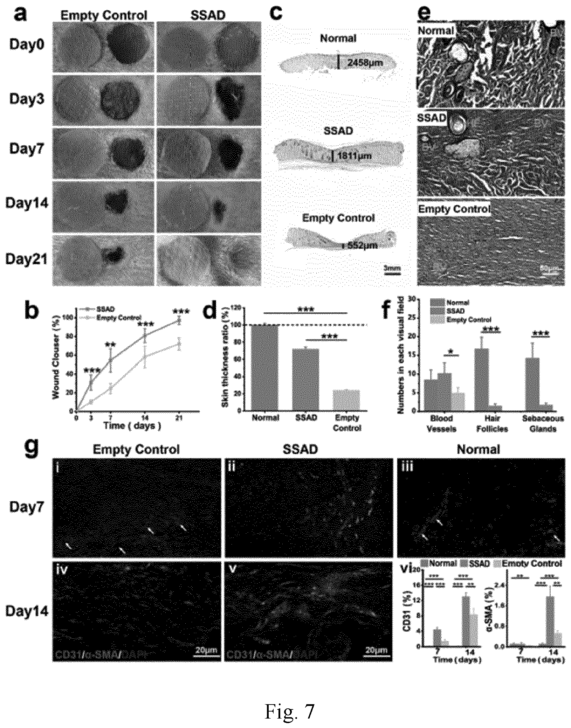

Fig. 7 shows the healing rate and quality evaluation of full-thickness skin gap wounds in diabetic SD rats of embodiment 5 of the present invention. Fig. 7a shows the visual observation of wound healing on days 3, 7, 14, and 21; Fig. 7b shows the results of statistical analysis of wound healing on days 3, 7, 14, and 21; Fig. 7c shows the histological observation of the epidermal healing of defect tissues in each group, wherein the black line is the test line of skin thickness (scale=3mm); Fig. 7d shows the statistical results of quantitative detection of skin thickness of each group of wounds; Fig. 7e shows the results of Masson staining of skin defects on the 21st day, and correspondingly mark hair follicles (HF), sebaceous glands (SG), and blood vessels (BV); Fig. 7f shows the number of blood vessels, hair follicles, and sebaceous glands in each field of view (scale=50$\mu$m); and Fig.7g shows angiogenesis-related markers CD31 and the staining results of $\alpha$- SMAi3 that can be used to determine the new shape of blood vessels at the wound site. (* P<0.05, ** P<0.01).

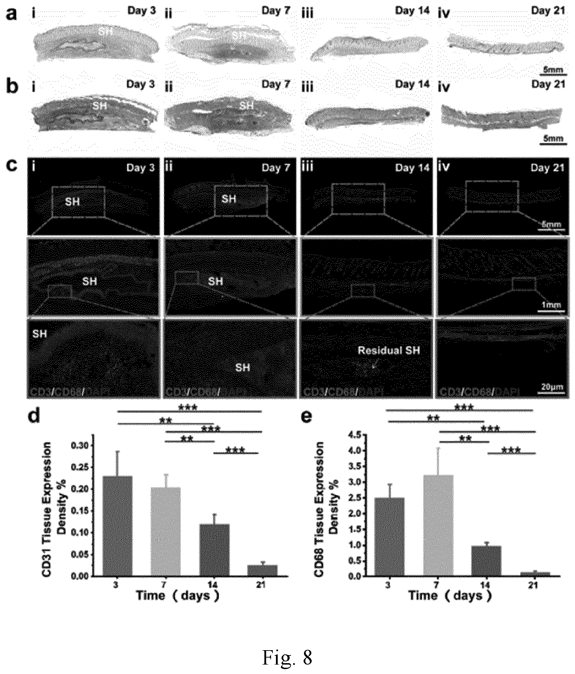

Fig. 8 shows the histological analysis of the degradation of the medical adhesive in vivo of embodiment 6 of the present invention.

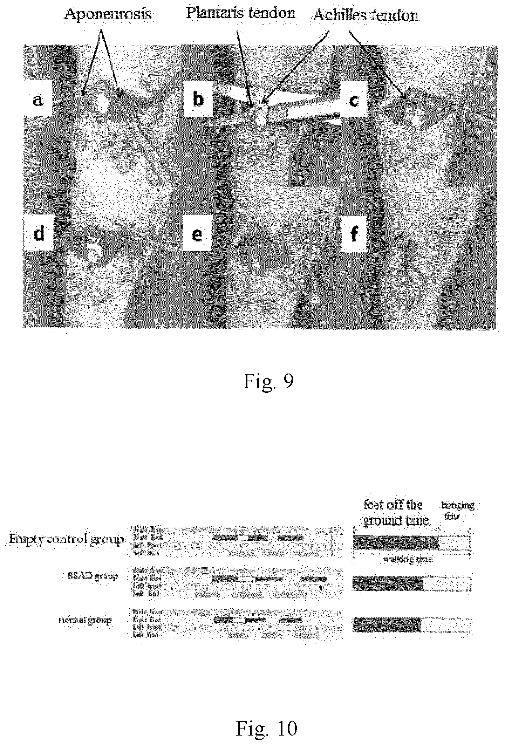

Fig. 9 shows the operation diagram of the rat Achilles tendon dissected defect model for evaluating the therapeutic effect of the medical adhesive on the tendon of embodiment 9 of the present invention.
Fig.10 shows the embodiment 9 of the present invention, which shows a comparison schematic of showing the maximum footprint length (the maximum length of the sole of the foot) of the SD rat, and the upper side (right leg) indicates the experimental leg, (compared with the normal control group, the footprint length is shorter, the healing is better).

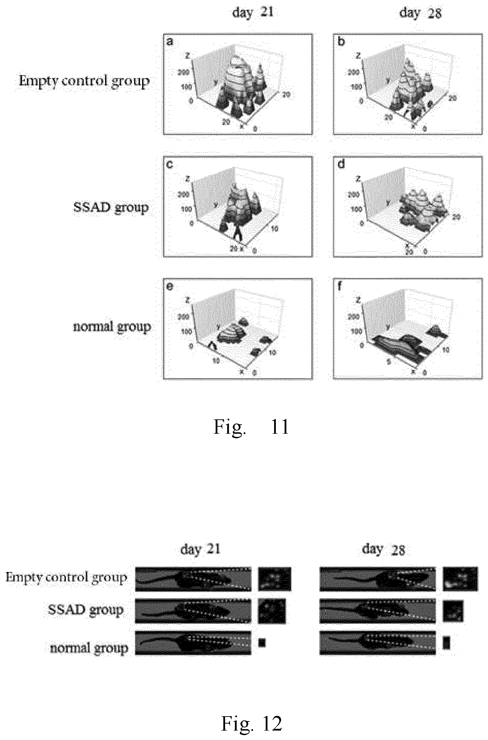

Fig. 11 shows the embodiment 9 of the present invention, the stride length (the distance between the midpoints of two consecutive footprints of the same foot) on the experimental side of the SD rat (compared to the normal control group, the step length is larger, the healing is better.)
Fig. 12 shows embodiment 9 of the present invention, the standing time of the experimental side in the walking cycle (refers to the time that the sole of the foot touches the ground in a walking cycle), the horizontal frame is wider, the contact time is longer (compared to the normal control group, the contact time is shorter, the healing is better).

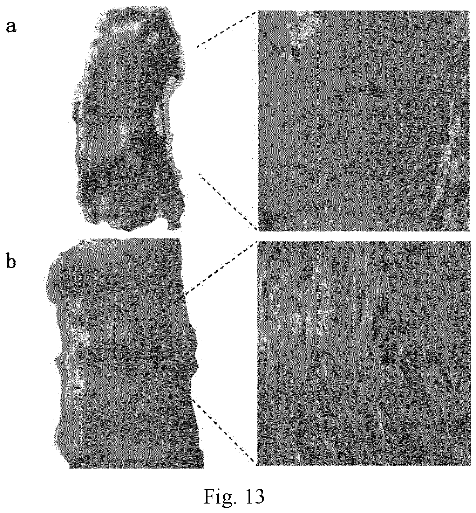

Fig. 13 shows embodiment 9 of the present invention that show HE stained photograph of the Achilles tendon section of the right hind leg (experimental side) of the rat after the operation for 28 days. wherein the collagen arrangement in the tendon was disordered in blank control group (a), and the cells had no obvious orientation; the collagen arrangement in the tendon was consistent and the cells had obvious orientation in the SSAD treatment group (b).

## DETAILED DESCRIPTION OF THE INVENTION

Some sample embodiments of the invention will now be described in greater detail. Nevertheless, it should be recognized that the present invention can be practiced in a wide range of other embodiments besides those explicitly described, and the scope of the present invention is expressly not limited except as specified in the accompanying claims.
The giant salamander skin mucus of originally collected is in gel form, which is difficult to sterilize thoroughly, inconvenient to store and is also difficult to use in clinical. In order to solve the above problems, the technical solution of the present invention is to provide a giant salamander skin mucus dried powder, which is sterilized and then is mixed with the aqueous solution to form a medical adhesive. The medical adhesive contains the ratio parts by weight of giant salamander skin mucus dried powder and aqueous solution is 1:1 to 1:6, that is, one part of giant salamander skin mucus dried powder is matched one to six parts of aqueous solution or (in the following description, the ratio parts by weight of the giant salamander skin mucus and aqueous solution is referred to "powder-to-water ratio"). According to the above preparation, the weight percentage of the giant salamander skin mucus freeze-dried powder in the medical adhesive ranges from 14.2% to 50%. The aqueous solution can be pure water or an aqueous solution containing biocompatible substance. In addition, the biocompatible substance mainly means that the aqueous solution prepared by adding biocompatible substance which is not to cause the active ingredients of the giant salamander skin mucus to be inactivated. Preferably, the aqueous solution is selected from the any one or more of the following: distilled water, deionized water, physiological saline (0.9% NaCl buffer), phosphate buffer (PBS), Tri buffer (TBS), citrate buffer, and chlorhexidine aqueous solution (preferably concentration is 2%) can be accepted for clinical. Moreover, the aqueous solution further includes human whole blood, and human blood extracts containing water, including plasma, serum, plasma, platelet-rich plasma (PRP), platelet-rich plasma fibrin (PRF). Base on the aspect of the present invention, blood or body fluid of human exuded from wounds can be used as the part of aqueous solution.

In addition to the giant salamander skin mucus dried powder and the aqueous solution as essential component, in order to manufacture the product (medical adhesive) that are easy to store, transport, and clinical applications, there are no limitation on the use of other component or raw materials to manufacture the appropriate medical adhesive. Specifically, according to the preferred embodiment of the present invention, the recipe of medical adhesive of the present invention also contains the other recipes that can stop bleeding, reduce inflammation or promote tissue growth, and it is not to limit herein.

In the present invention, the weight ratio between the giant salamander skin mucus dried powder and the aqueous solution, and the composition and the concentration for the aqueous solution are respectively designed, so that the medical adhesive has an ideal porous structure and good biocompatibility, and is suitable for adhesion of human body or other animal tissues, especially dense connective tissue, skin tissue, subcutaneous fat tissue, skeletal system or the adhesion between the aforementioned different types of tissues.

According to one of the preferred embodiment of the present invention, the steps of preparation method of the medical adhesive includes: obtaining mucus from a giant salamander skin, sterilizing and drying the giant salamander skin mucus, grinding the giant salamander skin mucus after sterilized and dried, and sieving the giant salamander skin mucus to obtain the particle size of the giant salamander skin mucus dried is met requirement, and used as one of the conditions for quality assurance. Next, the giant salamander skin mucus dried powder is sterilized and stored for later use.

The preferred technical solutions are described below based on the drawings. As shown in Fig. 1, the present invention provides a preparation method of the medical adhesive, the steps include: step 1: the mucus is obtained from the giant salamander skin, and the giant salamander skin mucus is frozen and dried. Step 2: the frozen and dried giant salamander skin mucus is crushed under frozen state, and then is sieved to obtain a powder with a specific particle size range. Step 3: the powder is sterilized to obtain a giant salamander skin mucus dried powder. Step 4: the sterilized giant salamander skin mucus dried powder is mixed with the aqueous solution according to the ratio of 1:1 to 1:6 to form a gel-form medical adhesive. Preferably, the mixing ratio of the giant salamander skin mucus dried powder and the aqueous solution is 1:2 to 1:6.

The collecting method of mucus in foregoing step 1 should be carried out in accordance with China's animal protection law, which can avoid the permanent disability of giant salamander without killing the giant salamander. The suitable collecting method can be scratching or electrical stimulation. Or commercially farmed giant salamander can also be killed to collect the mucus. It is not to limit herein.

In foregoing step 2, the collected giant salamander skin mucus dried is pulverized by a ball mill at a low temperature and then ground into a fine powder. In order to obtain the powder with a specific particle size (that is, the particle size does not exceed a specific specification), the different meshes sieves can be used for sieving.

Due to the slight difference in the international standards for the number of sieving meshes, the present invention preferably adopts the Taylor standard sieve system commonly used in Republic of China to define the number of sieving meshes. The division is based on 200-mesh sieve size of 0.074mm, the detailed calculation method is the conventional knowledge of the skilled in the art, and it is not to be repeated herein. However, when using above standard to describe the particle size of giant salamander skin mucus freeze-dried powder, the negative/positive symbol indicates whether the powder is passed through the mesh, in which the negative symbol indicates the mesh that the powder can pass through. That is, the particle size of powder is obtained after sieving is smaller than the mesh size. The positive symbol indicates the powder cannot pass through the mesh, which means the particle size of powder is obtained after sieving is larger than the mesh size.

Among then, in order to obtain the better performance of giant salamander skin mucus dried powder, the particle size of the giant salamander skin mucus freeze-dried powder is -20 meshes, which means the size of sieve mesh is larger than 20 mesh (the actual particle size obtained through experiments is approximately less than $850\mu m$). According to a preferred embodiment, the particle size of the giant salamander skin mucus freeze-dried powder is between -60 mesh and 300 mesh (the corresponding particle size is between $50$-$250\mu m$ obtained through experiments.) in a specific practice, -20 meshes of powder particle size is obtained with a sieve with mesh larger than 20 meshes. Usually, the particle size of the giant salamander skin mucus dried powder is not exceeding $850\mu m$ can be obtained.

After the step 2 is finished, the powder is stored in a refrigerator below -20 °C for later use.

In foregoing step 3, the powder obtained in step 2 is sterilized and disinfected. This step is an important step in applying the product for clinical and is related to the performance of the clinical. According to the relevant regulations, unsterilized and uninfected giant salamander skin mucus-related products cannot be directly used in clinical practice. Moreover, the mucus secreted on the surface of the living giant salamander may contain viruses or germs that are potentially harmful to human body which is collected in foregoing step 1. The viruses or germs cannot be inactivated thoroughly in step 2. If it is applied to the wound directly, the wound infection would be increased. The main components of giant salamander skin mucus are active ingredients such as proteins, peptides, mucopolysaccharides and antibacterial peptides. The best sterilization method will neither destroy or change the structure of biological macromolecules, nor decrease the adhesion performance and biological activity of the giant salamander skin mucus dried powder. According to current technology, the disinfectant sterilization method for giant salamander skin mucus-related product includes low temperature, ultraviolet rays, cobalt radiation, and disinfectant sterilization method. Preferably, the sterilization and disinfection in step 3 of the present invention is ethylene oxide sterilization method.

In foregoing step 4, the giant salamander skin mucus dried powder is mixed with the aqueous solution to form a gel, that is, the gel is the medical adhesive and the medical adhesive is in gel form. The aqueous solution includes but not limited to distilled water, deionized water and/or physiological buffer, such as physiological saline (NaCl buffer), phosphate buffer (PBS), Tris buffer (TBS), citrate buffer. The aqueous solution may also include 2% chlorhexidine, human whole blood, platelet-rich plasma (PRP), platelet-rich plasma fibrin (PRF), plasma and/or blood cell preparation, and above component can be used in combination as required.

According to the preferred embodiment of the present invention, the present invention further provides a use of the medical adhesive, specifically referring to the use in the adhesion, repair, and healing of wounds. Preferably, according to the performance of the medical adhesive of the present invention, especially suitable for the wound with dense connective tissue, specifically, the wounds includes but are not limited to the wound located in the epidermis, dermis and subcutaneous connective tissue. It should be emphasized that the medical adhesive is more particularly able to comply with the requirement of the wound adhesion in the skeletal and muscular systems. Therefore, the wound surface includes but are not limited to the wound located in skeletal muscle, tendon, ligament, bone, and/or the connective tissue around skeletal muscle.

Based on the above use, and further according to the preferred embodiment of the present invention, two methods are used as examples to illustrate the use of the medical adhesive, which includes but are not limited to.

Method 1: according to the hemorrhage and the body (especially human body) fluid exudate from the surface of the wound or wound, the giant salamander skin mucus dried powder and appropriate aqueous solution are directly applied to the wound surface to form a gel to achieve a better wound adhesion effect. When using the aqueous solution, more preferred method is to estimate the volumes of hemorrhage and body fluid of the wound surface or wound together, apply the appropriate aqueous solution and select the appropriate powder-to-water ratio, so the wound adhesion effect can be maintained. If the volume of hemorrhage and body fluid exudate from the wound or wound surface and the applied giant salamander skin mucus dried powder can be smoothly formed as a gel to achieve the better wound adhesion effect, so it is not necessary to additionally apply the aqueous solution to the wound or wound surface.

Method 2, 1 part of giant salamander skin mucus dried powder is mixed with 1-6 ratio parts by weight of aqueous solution form a gel of any shape, which can comply with the special shape requirement (such as cubic shape, surface area or thickness) to comply with the therapy requirement. For the wounds with larger hemorrhage or more tissue fluid exudation, an appropriate powder-to-water ratio is chosen to apply the gel to cover the wound to absorb the tissue fluid of the wounds to swell the gel, so the effect of the hemostasis, wet adhesion and the wound is maintained under drying condition.

The following examples illustrate the technical solution of the present invention and corresponding achieve effect.

Embodiment 1: Preparation of medical adhesive

Please refer to Fig. 2, as foregoing step 1, the living giant salamander is taken out (as shown in Fig. 2a), the giant salamander skin mucus is obtained by mechanical stimulation method (as shown in Fig. 2b), and the giant salamander skin mucus is collected for use later. Step 2, the collected giant salamander skin mucus is prepared to form dried powder (as shown in Fig. 2c) (white powder). In this procedure, the giant salamander skin mucus should be performed lyophilization procedure as soon as possible after collection, and the duration from the completion of collection to the dried powder is not exceed 1 hour (hereinafter referred to as "h"). The speed of lyophilization is set to drop 10°C -15 °C per hour, and the temperature will drop to -20 °C within 4 hours. Subsequently, the dried powder is pulverized or crushed to obtain the fine powder and is then sieved to remove the particle size of fine powder that is not comply with the requirement to obtain the dried powder whose particle size is not exceed a certain range. In this embodiment, the sieve with 14-mesh, 20-mesh, 60-mesh, 100-mesh, 200-mesh, and 300-mesh are used to sieve to obtain the particle size of freeze-dried powder is smaller than the diameter of the sieve aperture, and then the dried powder is placed in a closed container and is kept in refrigerator for later use.

Step 3, a sterilization treatment is performed with ethylene oxide to the dried powder. The dried powder is packaged in a sterilization packaging bag with ethylene oxide, or the sterilization package bag with ethylene oxide is packaged into a container with an opening and stuff a cotton ball into the opening of the container. The sterilization package bag is put into the ethylene oxide sterilization container and is sterilized by ethylene oxide. The sterilization time and temperature are based on the principles which is not destroy the performance of dried powder, it is not to limit herein. Subsequently, the sterilized dried powder is then stood to volatilize the residual ethylene oxide in the freeze-dried powder. Final, seventh part of "GB/T 16886.7-2001, Biological evaluation of medical device" is referred: evaluation of residual amount of ethylene oxide sterilization to estimate the ethylene oxide residual" to inspect the ethylene oxide remained in the freeze-dried powder. After passing the inspection, the overall sterilization step is completed.

In addition, in order to illustrate the effect of using ethylene oxide in the present invention, there are several different sterilization methods to evaluate, the described as follows. First, for the evaluation of possible sterilization methods, the sterilization effect of low temperature and ultraviolet light is suspected to incomplete sterilization. The sterilization duration is long, and the giant salamander skin mucus freeze-dried powder has been sterilized under low temperature and ultraviolet light that is easy to hydrolysis and the expiration date is shorter. The Gamma-ray method involves more complicated operation due to the radiation, and it will reduce the adhesion performance of the related product of giant salamander skin mucus.

For the ethylene oxide sterilization method proposed in the present invention, it is preferable to refer to the National standard GB18279-2000 "Ethylene oxide sterilization confirmation and regular control of medical device" to perform the sterilization step: the giant salamander skin mucus freeze-dried powder is crushed to package into ethylene oxide sterilization package bag, or put into a container with an opening, and then a cotton ball is stuffed to opening of the container. By using the ethylene oxide, the sterilization parameter is 100% ethylene oxide, temperature is at 54 °C, the sterilization time for 60 minutes, and analysis for 15 hours. After sterilizing, seventh part of "GB/T 16886.7-2001 "Biological evaluation of medical device" is referred: evaluation of residual amount of ethylene oxide sterilization to estimate the residual of ethylene oxide" to inspect the ethylene oxide remained in the freeze-dried powder, the residual amount of ethylene oxide in the giant salamander skin mucus freeze-dried powder is smaller than 10ppm. After qualifying the inspection, the entire sterilization steps are complete.

According to the clinical safety requirements, the comparison results of the sterilization performance of above ethylene oxide sterilization method and other sterilization methods are shown in Fig. 3C. The results show that the ethylene oxide sterilization and cobalt ray sterilization, the sterilization effect is better than the low temperature sterilization (includes -20°C, -50°C, -80°C and liquid nitrogen) and ultraviolet light (as shown in Fig. 3C-a). The cobalt ray sterilization has a greater impact on the adhesion. The ethylene oxide sterilization method is capable of good adhesion performance (as shown in Fig. 3C-b) and is the best sterilization and disinfection method for the giant salamander skin mucus dried powder.

Unless otherwise specified, the dried powder (usually abbreviated or labeled as "SSAD") used in the following examples are all obtained through above steps.

As foregoing step 4, the giant salamander skin mucus dried powder has passed the inspection which is mixed with the aqueous solution to form a gel-form medical adhesive. The ratio parts by weight of giant salamander skin mucus dried powder and aqueous solution is 1:1 to 1:6, and the preferred ratio parts by weight is 1:2 to 1:6, that is, one part by weight of giant salamander skin mucus dried powder mixed with 2-6 parts by weight of aqueous solution. In the gel-form medical adhesive of the present invention, the preferred weight percentage of the giant salamander skin mucus dried powder ranges from 14.2% to 50%.

The design of weight ratio of giant salamander skin mucus dried powder and the aqueous solution of the present invention is to give the special, practical and excellent gel performance. Specifically, when the giant salamander skin mucus dried powder is mixed with aqueous solution, the polypeptide crosslinking network swells instead of dissolving. After mixing, the entangled protein network of giant salamander skin mucus dried powder is changed to drive by hydrogen bonds, disulfide bonds, and $\pi$-$\pi$ conjugated bonds to form gel (as shown in Fig. 2d and Fig. 2e). This procedure is called "hydrogel formation". During hydrogel formation, the amino acid residues of the polypeptide chain undergo a conformational transformation to form a gel-form medical adhesive. The phenolic hydroxyl and amino groups are used as a hydrogen bond donor to transform to a high surface energy or hydrophilic interface transformation, so the biological adhesion through the Van der Waals force is promoted. In addition, the benzene ring is interacted drastically with the substrate through $\pi$-$\pi$ electrons or cation-$\pi$ interaction when the benzene ring contacts low surface energy or hydrophobic interface.

Based on above characteristics of the medical adhesive of the present invention, in view of the fact that wound interface of human or animal is protein-rich extracellular, thus, it is necessary to select an appropriate powder-to-water ratio for preparing the gel, the synergistic effect between active substance, aqueous solution in the medical adhesive and the intercellular fluid in wound tissue to make the medical adhesive and the wound can form the best adhesion through the hydrogen bond and Van der Waals force.

According to above, the pure water or clinically acceptable aqueous solution can be selected for the aqueous solution, which can contain biocompatible substances. The selection of biocompatible substances will not cause the active recipes in the giant salamander skin mucus and does not affect the gelation. Therefore, except for the aqueous solution is artificially prepared, the blood or tissue fluid exuded from the wound surface or wound are also used as the aqueous solution to mix the foregoing aqueous solution to form aqueous solution.

In addition, because surgical wound surface and wound are usually located at the interface between the body surface and the outside, and at the same time, when the external temperature change and the constant temperature of human tissue at about 37°C is faced, good performance is a requirement for hydrogel formation. Therefore, in one embodiment, the hydrogel formation of medical adhesive is evaluated at 4°C or 37°C. 100mg of giant salamander skin mucus dried powder is mixed with 200μL PBS (aqueous solution) to form a mixture, and then the mixture is poured into a mold to stand at 4°C or 37°C for more than 3 minutes, until the mixture is in gel form which used as medical adhesive. It can be shown from the experimental, the giant salamander skin mucus dried powder and aqueous solution can quickly form a viscously gel.

Embodiment 2: Structural analysis of medical adhesive

The medical adhesive provided by the present invention has porous structure therein after hydrogel formation, so the porous structure of the medical adhesive can exchange the nutrients in tissue fluid and the metabolites during clinical application.

The scanning electron microscope (Hitachi, S-3400N II, Japan) analyzes the porous structure of giant salamander skin mucus dried powder (SSAD powder) with different particle size that obtained in embodiment 1 of the present invention, and porous structure of giant salamander skin mucus dried powder shows the uniform block scanned by scanning electron microscope as shown in Fig. 3A. However, when the giant salamander skin dried powder mixed with the aqueous solution, the polypeptide chains in the block will swell and penetrate each other to form a gel-form medical adhesive (SSAD) with a three-dimensional honeycomb structure.

According to the analysis of the scanning electron microscope, the average diameter of the porous structure of three-dimensional honeycomb structure decreased with the decrease of the particle size of giant salamander skin mucus dried powder. It has not been found that the powder-to-water ratio will affect the average diameter of porous structure, and there is not statistically significant for the correlation between the average diameter of porous structure and the powder-to-water ratio. The giant salamander skin mucus dried powder with a particle size of -14 mesh is hydrated to form a gel which has average diameter of porous is 116 microns (μm), the average diameter of porous for particle size of -60-mesh is 37μm, and the average diameter of porous for the particle size of -300-mesh is 6μm. In addition, as the increase of gelation time, the porous structure becomes more obvious, and the porous size becomes more uniform, in the porous structure after gelation for 12 hours, the sidewall density of cavity is increased (gelation time is 12 hours), which is larger than sidewall after gelation for 2 hours (gelation time is 2 hours). It shows that with the hydration time is increased, the structure of porous network tends to be stable. The result of analyzing the porous diameter of porous structure and the porous size is shown in Fig. 3B.

Embodiment 3: in vitro determination of the tissue adhesive intensity of medical adhesive

The testing in this embodiment (embodiment 3) is in accordance with ASTM (American society for Testing and Materials), the commercially available medical adhesive cyanoacrylate and fibrin is used as blank control group to evaluate the adhesion effect. The improvement is made in response to different materials and operating environment which should be understandable and acceptable to above skilled in the art, and it will not be repeated herein.

In this embodiment, the laboratory shear test is performed with universal testing machine (MTS Criterion, Model 43, USA). The specific method is to use the pig skin as a tissue matrix, the pig skin is cut into 1x8 square-center meter rectangles. By using cyanoacrylate (Baiyun Medical Adhesive®, Guangzhou, China) (as labeled "commercial gel" in Figure), fibrin gel (FIBINGURAAS®, Shanghai, China) (as labeled "bio gel" in Figure), and the medial adhesive is made by giant salamander skin mucus dried powder (as labeled "SAAD treatment group, there are two adhesion methods: one is incision aligning adhesion (incision edge is faced to the incision edge), and the other is subcutaneous fat adhered to the subcutaneous fat, the practice and result are shown in Fig. 4A and Fig. 4B respectively.

For SSAD treatment group, the specific testing method is to take 30mg by weight of giant salamander skin mucus dried powder to apply on pig skin, and adds 60μL-180μL of PBS (ratio parts by weight is about 1:2 to 1:6) by using pipette to form medical adhesive. On the other hand, the bio gel group and commercial gel group of control experiment respectively used fibrin and cyanoacrylate to adhere pig skin in accordance with above two adhesion methods. After adhering for two hours, the performance of adhesion parts is tested on universal testing machine.

The lap shear testing is used to determine the shear intensity of materials of above three groups according to ASTM F2255-05 standard. For anti-shearing adhesion ability of incision alignment adhesion method (as shown in Fig. 4A-i), the adhesion intensity is 40.71±3.71kPa for the commercial gel group and the adhesion intensity is 26.66±8.22 kPa for the SSAD treatment group (as shown in Fig. 4A-ii and Fig. 4A-iii). The result shows that, compared the different between bio gel group and commercial gel group, the adhesion intensity of SSAD treatment group (P<0.05) and adhesion intensity of commercial gel group (P<0.01), the comparison results are statistically significant. The testing result of bio gel group showed that low adhesion ability of fibrin gel complied with its use instructions: fibrin gel is not used alone but with the sutures. It should be noted that the result of lap shear testing showed that fracture strain of the commercial gel group much higher than that of SSAD treatment group. During lap shear testing, part of epidermis was damaged, which indicates that adhesion ability of cyanoacrylate adhesive to skin adhesive is too high to cause the negative effect of skin damage. In addition, although the adhesion ability of commercial gel group is strongest among three groups, but the commercial gel group only adhered to epidermis but not for subcutaneous fat.

In contrast, when the subcutaneous fat method is used to adhere to subcutaneous fat (as shown in Fig. 4B-i) to adhere pig skin, the adhesion ability of SSAD treatment group is significantly better than commercial gel group and bio gel group. As shown in Fig. 4B-ii and Fig. 4B-iii, the adhesion ability of SSAD treatment group has significantly anti-shear ability in adhesion ability of subcutaneous fat, the shear adhesion force is about 26.11±7.72kPa, and the shear adhesion force for other two adhesives are smaller than 7kPa.

The bio gel adhesive not only has good adhesion abilities, but also has good elasticity and ductility. Therefore, except for the adhesion force, this example further performs elasticity and ductility for above material of three groups, the results are respectively shown in Fig. 4C. The specific testing method is to use pig skin as the biological matrix and adopt the three-points adhesion testing which is modified by three-points bending method to determine the elasticity and ductility of pig skin after adhesion. The three-points adhesion testing is to make a 2cm incision in the middle of pig skin (as shown in Fig. 4C-i). Using force transducer with 100N is loaded and separation speed of 1mm/min to separate the adhesion sample completely to detect the stress is to be loaded and is required for causing the same displacement, so the representative schematic of strain-displacement curve (as shown in Fig. 4C-ii) and load quantitative required for fixed deformation (as shown in Fig. 4C-iii). The testing results of comparison of above materials of three groups, when the same deformation is achieved, SSAD treatment group only needs 7.84±1.17N, which is similar to bio gel group (7±0.99N), but significantly lower than commercial gel group (12.33±1.53N) (Statistical differences shown in Fig. 4A, Fig. 4B, and Fig. 4C: * P<0.05, ** P<0.01, *** P<0.00 1).

The testing results show that cyanoacrylate adhesive provides rigid adhesion, while the medical adhesive of the present invention can provide a flexible adhesion to skin, which indicates the adhesion ability of medical adhesive of the present invention are more suitable for skin and tissue adhesive.

The above adhesion ability, anti-shearing, elasticity, and ductility abilities of testing pig skin is compared with two medical adhesives (cyanoacrylate synthetic gel and fibrin gel) which commonly use in clinical. The overall results show that the medical adhesive provided by the present invention exhibits superior adhesion ability on difference substrates.

In order to test the adhesion ability of medical adhesive of the present invention, this example utilizes universal testing machine (MTS Criterion, Model 43, USA) to perform laboratory shear testing. The specific method uses bone as the tissue matrix, a rabbit femur is taken to cut at the middle by using bone scissors, and the medical adhesive is made of giant salamander skin mucus freeze-dried powder to perform adhesion contraposition for the cut rabbit femur . After adhering for 10 mins, the shear testing is performed to obtain the adhesion intensity is 38.5±11.2KPa.

In vitro testing and comparison are carried out for the abilities (such as bacteriostatic effect, anti-oxidation effect, and cell migration effect) of medical adhesive of the present invention applied for wound surface or wound, and the following experiments are used to illustrate.

Experiment 1: bacteriostatic effect experiment

The giant salamander skin mucus extraction solution used for aerobic bacteria experiment is prepared as following: 1g by weight of sterile giant salamander skin mucus dried powder is taken, an appropriate amount of LB medium nutrient broth medium) is added to make the giant salamander skin mucus dried powder fully absorb the LB medium to swell. Then, the culture medium is continuously added until the amount of liquid culture medium reached 10mL. Next, the liquid culture medium was placed in a refrigerator at 4°C for 24 hours to complete the extraction procedure.

The giant salamander skin mucus extraction used for anaerobic bacteria experiment is prepared as following: 1g by weight of sterile giant salamander skin mucus dried powder is taken, an appropriate amount of TSB medium (tryptone soy broth medium) is added to make the giant salamander skin mucus dried to absorb the TSB medium to swell. Then, culture medium is continuously added until the amount of liquid culture medium reached 10mL. Next, the liquid culture medium was placed in a refrigerator at 4°C for 24 hours to complete the extraction procedure.

The aerobic bacteria are *Escherichia coli* and *Staphylococcus aureus.* The culture method is described as follows: the extraction solution is prepared for aerobic bacteria experiment. After extracting for 24 hours, the extraction solution is centrifuged at 3000 r/min for 3 minutes to obtain the final extraction solution (also called as giant salamander skin mucus extraction solution). Next, 10mL of giant salamander skin mucus extraction solution and 500 of bacterial solution are respectively added into 50mL centrifuge tube. Next, after giant salamander skin mucus extraction solution and bacterial solution are mixed well in centrifuge tube, the centrifuge tube (contains giant salamander skin mucus extraction solution and bacterial solution therein) is put into incubator immediately.

*Porphyromonas gingivalis* is selected as anaerobic bacteria, and the culture method is described as follows: the extraction solution is prepared for the anaerobic bacteria experiment. After extracting for 24 hours, the extraction solution is centrifuged at 3000 r/min for 3 minutes to obtain the final extraction solution (also called as giant salamander skin mucus extraction solution). Next, 10mL of giant salamander skin mucus extraction solution and according to 5% concentration of sheep blood, 500$\mu$L of sheep blood is added into each centrifuged tube with giant salamander skin mucus extraction solution. Next, 500$\mu$L of bacterial solution with same contraction as sheep blood is then incubated and is mixed well. Then, the centrifuged tube (contains giant salamander skin mucus extraction solution, 500$\mu$L, 5% concentration of sheep blood and bacterial solution) is immediately put into the anaerobic incubator.

The blank control group is prepared as follows. Aerobic bacteria: 10 mL of culture medium is added into centrifuge tube. The concentration of bacterial solution is to be incubated which is same as that of the experimental group as blank control group. Anaerobic bacteria: 10mL of TSB cultural medium and 500$\mu$L of sheep blood are respectively added into centrifuge tube. The bacterial solution is to be incubated which is same as the experimental group as blank control group.

The bacteria detection method is described as follows. The detection of *Escherichia coli* and *Staphylococcus aureus:* the absorbance is determined by microplate absorbance reader (EnSpire, PerkinElmer, Singapore). The operation method is that from 0th hour, every 2 hours, 100$\mu$L of mixed well solution per well is drawn to add into 96-well plate, and three secondary wells are disposed. Under the condition OD (optical density) is 600, the absorbance of each group is to be determined. The data are shown in table 1 and table 2.

Detection of *Porphyromonas gingivalis:* the absorbance is determined by microplate absorbance reader. The operation method is that from day 0, 100$\mu$L of mixed well solution per well is drawn to add into 96-well plate, and five secondary wells are disposed. Under the condition OD is 600, the absorbance of each group is to be determined. The data are shown in Table 3.

## Table 1: Comparison of antibacterial effects of *Escherichia Coli*

| Experimental material / Sampling duration | Giant salamander skin mucus extraction solution | Blank control group |
|---|---|---|
| 0h | 0 | 0 |
| 2h | 0.018±0.013 | 0.031±0.002 |
| 4h | 0.016±0.005 | 0.052±0.016 |
| 6h | 0.014±0.007 | 0.073±0.004 |
| 8h | 0.013±0.006 | 0.108±0.007 |
| 10h | 0.036±0.003 | 0.145±0.005 |
| 12h | 0.048±0.003 | 0.164±0.008 |
| 14h | 0.078±0.007 | 0.181±0.001 |
| 16h | 0.092±0.007 | 0.209±0.002 |
| 18h | 0.121±0.004 | 0.221±0.002 |
| 20h | 0.148±0.006 | 0.232±0.003 |

## Table 2 Comparison of antibacterial effects of *Staphylococcus Aureus*

| Experimental material / Sampling duration | Giant salamander skin mucus extraction solution | Blank control group |
|---|---|---|
| 0h | 0 | 0 |
| 2h | 0. 003±0. 002 | 0. 068±0. 002 |
| 4h | 0. 006±0. 004 | 0. 084±0. 004 |
| 6h | 0. 005±0. 003 | 0. 105±0. 002 |
| 8h | 0. 019±0. 004 | 0. 186±0. 010 |
| 10h | 0. 043±0. 002 | 0. 196±0. 004 |
| 12h | 0. 050±0. 004 | 0. 207±0. 002 |
| 14h | 0. 079±0. 003 | 0. 221±0. 003 |
| 16h | 0. 093±0. 003 | 0. 243±0. 003 |
| 18h | 0. 109±0. 002 | 0. 253±0. 002 |
| 20h | 0. 109±0. 001 | 0. 274±0. 022 |

Table 3 Comparison of antibacterial effects of *Porphyromonas gingivalis*

| Experimental material / Sampling duration | Giant salamander skin mucus extraction solution | Blank control group |
|---|---|---|
| 0day | 0 | 0 |
| 1day | 0. 073±0. 015 | 0. 107±0. 008 |
| 2day | 0. 142±0. 042 | 0. 176±0. 017 |
| 3day | 0. 181±0. 057 | 0. 212±0. 016 |
| 4day | 0. 241±0. 005 | 0. 275±0. 042 |
| 5day | 0. 264±0. 053 | 0. 361±0. 013 |

The above experiments prove that the giant salamander skin mucus extraction solution has obvious inhibitory effect on two common aerobic bacteria (*E. coli and Staphylococcus aureus*), the anti-bacterial time can reach 16 hours. The giant salamander skin mucus extraction solution has significant inhibitory effect on *Porphyromonas gingivalis* (anaerobic bacteria), and the antibacterial time can reach more 5 days.

Experiment 2: antioxidant experiment

First, the giant salamander skin mucus extraction solution is prepared. The method is described as follows: 200mL of giant salamander skin mucus dried powder is added into 2ml of deionized water (weight ratio is about 1:10) for 7 days to make the soluble substance of giant salamander dried powder dissolved thoroughly. The supernatant is extracted from the extraction solution of giant salamander skin mucus. In addition, 1mg/mL of DPPH ethanol solution is prepared on site.
The experiment group utilizes 1mL of giant salamander skin mucus extraction solution and 1mL of DPPH ethanol solution. The blank control group utilizes 1mL of pure water and 1mL of DPPH ethanol solution. Next, the spectrophotometer is calibrated to zero with ethanol, and the wavelength of experimental group and blank control group are measured at a wavelength of 517nm at 0.5 hours, 1 hour, and 2 hours respectively. The absorbance value of blank control group indicates $A_1$ and the absorbance value of experimental group indicates $A_2$. The calculation formula of free radical scavenging rate is formula (I), and the measurement of absorbance for blank control group and experimental group are shown in Table 4.

$$\text{Free radical scavenging rate} = 100 \times (A_1 - A_2) \div A_1 \text{ (formula (1))}$$

Table 4 calculation table of Free radical scavenging rate

|  | blank control group ($A_1$) | experimental group ($A_2$) | Free radical scavenging rate |
|---|---|---|---|
| 0.5h absorbance value | 0.461 | 0.106 | 77% |
| 1h absorbance value | 0.451 | 0.099 | 78% |
| 2h absorbance value | 0.441 | 0.082 | 81.4% |

This experiment shows that the giant salamander skin mucus extraction solution can scavenge most of free radicals in DPPH solution, and the giant salamander skin mucus extraction solution has antioxidation ability.

Experiment 3: cell migration experiment

In order to simulate the wound healing process in vitro, the cell scratch experiment and transwell chamber experiment are carried out.

For above two experiments, the fully medium is first prepared: 88 parts of DMEM, 10 parts of fetal bovine serum, 1 part of penicillin and 1 part of streptomycin are taken and are mixed to obtain the fully medium. Next, according to the ratio of 1mg of giant salamander skin mucus dried powder to 1mL of fully medium, the giant salamander skin mucus freeze-dried powder is soaked in the fully medium for 7 days to fully dissolve the soluble substance in giant salamander skin mucus to obtain the concentration of 1mg/mL of giant salamander skin mucus extraction medium. Further, 5 different concentration ratio of giant salamander skin mucus extraction medium is prepared, the concentrations are 0.5mg/mL, 0.1mg/mL, 0.05mg/mL, 0.01mg/mL, and 0.005mg/mL respectively.

In the cell scratch experiment, HUVES and L929 cells are performed for testing. The cell preparation process is: HUVES (umbilical vein endothelial cells) and L929 (fibroblasts) in the logarithmic growth phase are taken, trypsin digestion to obtain a single cell suspension, the cell count of $6 \times 10^5$ cell /mL. 1mL of cell suspension is inoculated into 6-well plate and 1mL of fully medium is added and cultured in an incubator at 37°C. After the wall is overgrowth with 6-well plate, 200 of yellow pipette tip to make scratch (scratch in horizontal and vertical to make the scratch "+" for observing). The scratch "+"is then washed with PBS for 2-3 times to remove the floating cells. Finally, 2mL of 5 different giant salamander skin mucus extraction mediums are added into 5 well, and fully medium without giant salamander skin mucus extraction medium is added into one well which used as blank control group. All the experimental samples are cultured in 37°C incubator and is observed and photographed under 0 hour and 24 hours respectively, in which the experimental results of 0.1mg/mL of giant salamander skin mucus extraction medium as represented, as shown in Fig. 5A, and remaining concentrations are not shown herein.

By using Image-J software to calculate and analyze the area of collected pictures, the results is shown in Fig. 5B. Experiments have proven that different concentration of giant salamander skin mucus extraction medium can promote the migration of HUVES and L929 cells, in which the concentration of 0.1mg/mL has the most significant effect, and the cell growth and migration is faster than that of the blank control group obviously and it has statistically significant in difference between experimental group and blank control group. Then, the transwell chamber experiment was carried out to further prove 5 different concentration of giant salamander skin mucus extraction medium effect on the migration of HUVES and L929 cells in vitro to reflect the role in promoting skin and mucus membrane regeneration.

HUVES and L929 cells are respectively performed for testing, and the cell preparation process is: HUVES and L929 in logarithmic growth phase are taken, and trypsin digestion to obtain a single cell suspension, $2 \times 10^5$ cell/mL of the cell count HUVES and $3 \times 10^5$ cell/mL of cell count L929. The transwell chamber is placed in 24-well plate, and then 100μL of cell suspension is inoculated into the upper chamber of transwell, and 800μL of giant salamander skin mucus extraction medium respectively added into lower chamber to incubate in 37°C incubator for 24 hours. After incubating for 24 hours, the chamber is taken out of the incubator, the fixed liquid of upper chamber is soaked, and the chamber is pre-moved to the well of 800μL of methanol. The chamber is taken out of incubator after temperature of incubator is kept in 30 mins, the liquid of upper chamber is blot and the chamber is moved to 24-well plate where pre-added with 800μL and concentration is 0.1% of crystal violet stain solution, stain at room temperature for 15-30 minutes. After staining, the PBS is soaked several times to remove the excess stain solution. Finally, the liquid in upper chamber is soaked, the cell on the surface of the membrane at bottom of upper chamber is removed by cotton swab. After chamber membrane is dried, the membrane is tore-out by tweezers and is placed on the microslide and is covered by cover glass to observe under 200X microscope, he results as shown in Fig. 5C. Then, the cells are counted on the photo taken randomly from the samples of each experimental group, 5 fields of view are randomly selected for each sample. Fig. 5C shows 1 field of view of each sample. The number of cells obtained by counting is shown in Table 5. Finally, the obtained number of cells is performed difference statistical analysis.

## Table 5: transwell chamber experimental results

| medium / cell | blank control group | 0.5mg/mL | 0.1mg/mL | 0.05mg/mL | 0.01mg/mL |
|---|---|---|---|---|---|
| HUVES | 77±6 | 145±15 | 227±18 | 72±6 | 72±5 |
| L929 | 108±8 | 109±13 | 223±7 | 152±12 | 127±11 |

The experiment results prove that different concentration of giant salamander skin mucus extraction medium have different effects on the migration rate of two kinds of cells, in which, for HUEVS, it has statistically significant in difference between 0.5mg/mL, 0.1mg/mL of giant salamander skin mucus extraction medium and blank control group; for L929, it also has statistically significant in different between 0.1mg/mL, 0.05mg/mL, and 0.01 mg/mL of giant salamander skin mucus extraction medium and blank control group, in which 0.1mg/mL of giant salamander skin mucus extraction medium has the most significant promotion effect on two kinds of cells. The experimental results show that different concentration of giant salamander skin mucus extraction medium have different effects on migration rate of two kinds of cells, and the promotion effect of 0.1mg/mL of giant salamander skin mucus is particularly significant.

The in vitro cell experiment results show that the cell compatibility of giant salamander skin mucus extraction medium is better, it no obvious toxicity for cell, and 0.1mg/mL of giant salamander skin mucus extraction medium can significantly accelerate the migration of HUVES and L929.

Experiment 4: Evaluation and effect of medical adhesive on tissue adhesion of wound of living body

It should be noted that the animals used in the experiment of the present invention are all purchased from the experimental animal center of ChongQing Medical University. All animal studies are conducted in accordance with NIH guidelines on the care and use of laboratory animals (NIH Publication No. 85-23 Rev. 1985) and are approved by Animal Care and Use Committee of the School of Dentistry Chongqing Medical University (CQHS-REC-2018-01).

For evaluation of in vivo wound adhesion and biocompatibility, 30 male Sprague-Dawley (SD) rates (6-8 weeks old rats, the weight is 200g±20g) are used in experiment. SD rats are anesthetized by intraperitoneal injection of sodium pentobarbital (30mg/kg). After shaving the back hair, the SD rats is disinfected with iodine and ethanol. The back of SD rats is cut to 4 incisions and each incisions is 2cm, and the treatment utilizes 4-0 non-absorbable sutures is used to suture the wound, the medical adhesive (SSAD) of the present invention, cyanoacrylate (commercial gel), fibrin gel (bio gel) or hemostasis (blank control group).

For SSAD treatment group, 5mg of giant salamander skin mucus dried powder applied to the incision, and then 15μL-20μL of PBS applied to incision, and wound edges are pressed each other to contact for about 30 seconds, so the giant salamander skin mucus dried powder interacted with PBS to enable the adhesion effect. The results show that cyanoacrylate used in commercial gel group has rigid adhesion properties compared to medical adhesive used in SSAD treatment group, and the medical adhesive has the flexibility as same as normal skin. In addition, although fibrin gel in bio gel group also has a certain flexibility, but the low adhesion effect to cause the dehiscence of adhering incision during moving, the adhesion ability of bio gel group is still inferior to that of the medical adhesive.

All wounds are taken a picture and observed to understand the healing procedure and monitor the changes in wound healing condition (as shown in Fig. 6a). After operation for 5 days, the wounds of blank control group that is not performed with sealing treatment are dehisced (as shown in Fig. 6a). comparing bio gel group and blank control group (only hemostatic treatment), commercial gel group, and suture group, all wounds in SSAD treatment group are recovered, in which the wounds in SSAD treatment group not only recovered but there is no scars, and the healing and recovery effect better than other experimental groups. Furthermore, there is no suspicious signs of infection or inflammation wraparound the wounds for SSAD treatment group, so the medical adhesive of the present invention exhibits the superior effect on wound healing.

To evaluate side effect of rat skin tissue which may cause by the wound healing effect and medical adhesive of the present invention, the rats are sacrificed after operation for 5 days. the skin sample (3x3cm) is collected for hematoxylin and eosin staining analysis (H&E; G1120, Solarbio, China), to perform a histological studies, the results as shown in Fig. 6b. In SSAD treatment group, it can be seen longitudinal collagen fibers, scattered neutrophils and fibroblasts below the incision is treated by medical adhesive. The epithelium and substrate membrane are continuously fused, and there are no cracks deeply in the tissue. In addition, hair regrowth is observed at the incision without scars, so the medical adhesive can promote the overall wound healing without other side effect obviously.

The groups other than SSAD treatment group, there are some irregulate collagen fibers, neutrophils and fibroblasts at the suture wound tissue, and there are less hair regeneration, it is worth noting that compared with SSAD treatment group, there are no cells in the sutured incision site in other groups, while the SSAD treatment group is full of new cells of stained with nuclear blue. In commercial gel group, the incision is treated with cyanoacrylate, it can be obviously to look out the vacuole wraparound the bottom of incision, and nuclear blue stained cell can be seen obviously in the cell area of substrate. The incision of bio gel group obviously showed the ulcer surface and the undegraded fibrin gel is residue at the bottom. The hemostatic treatment is only performed in the blank control group, the incision is filled with a large amount of granulation tissue, and a certain number of polymorphonuclear leukocytes, macrophages, fibroblasts and blood capillaries cab be seen in the wound region.

The cyanoacrylate in the commercial gel group has the highest adhesion performance in vivo and in vitro, even higher than the intensity of natural skin tissue. However, the adhesion performance of cyanoacrylate to fat is weak, the adhesion interface is more rigid, and the cytotoxicity is not to be ignored. The fibrin gel in bio gel group has similar properties to soft tissue, but the adhesion performance is low and cannot be used alone. As for the effect of SSAD treatment group, the recovery of treated incision is better than that of the normal sutured incision, and there is no wound infection or inflammation obviously and has the effect of promoting cell regeneration.

Experiment 5: Evaluation of effect of medical adhesive on wound healing in vivo

This experiment is applied for full-thickness skin defect to evaluate the healing ability of medical adhesive applied to wound in diabetic SD rats. According to the method described in paper (Biomaterials science. 2018;6: 2757-72), after the diabetic rats are constructed successfully, the diabetic rats are anesthetized with 1% sodium pentobarbital (intraperitoneal injection), and the hair of the back of diabetic rats are scraped. By using a disposable biopsy needle to make full-thickness circular wound with a diameter 10mm on the back skin of diabetic rats. In wound treatments, the blank control group utilizes the gauze to cover the wound (fully-thickness circular wound with a diameter 10mm); SSAD treatment group calculates the amount of giant salamander skin mucus dried powder according to $30mg/cm^2$ defect area, and the giant salamander skin mucus dried powder is spread over the wound surface, and the animal is kept in a static state for more than 2 minutes, so that the giant salamander skin mucus dried powder absorbs the blood of the wound (that is, the function of an aqueous solution) to form a gel which is a medical adhesive to cover the wound. After operation for 0, 3, 7, 14, and 21 days, the wound sealing rate is to be analyzed; after operation 21 days, the wound is performed with tissue section analysis which includes STR (skin thickness ratio), average normal skin thickness and skin appendages (such as hair follicles, sebaceous glands, sweat glands) for statistical analysis.

The wound healing ratio is calculated by using Image J software (National Institute of Health) according to the following formula (Formula (II)):

$$wound\ sealing\ ratio = \frac{S_{initial} - S_{current}}{S_{initial}} \times 100\% \quad (Formula\ (II))$$

$S_{initial}$ indicates the initial wound size, $S_{current}$ indicates the current wound size. Each condition must be tested at least three times.

The skin thickness ratio (STR) is calculated by using Image J software according to formula (III):

$$skin\ thickness\ ratio = \frac{T_{scar}}{T_{Normal}} \times 100\% \quad (Formula\ (III))$$

$T_{scar}$ indicates average skin thickness of scar tissue, $T_{Normal}$ indicates average normal skin thickness. Each condition must be tested at least three times.

In order to evaluate efficiency of medical adhesive applied on wound in vivo, by using a disposable biopsy puncture machine to make 1cm of a round full-thickness skin wound on the back of rats. The SSAD treatment group utilizes the medical adhesive as prepared in experimental 1 and the amount of medical adhesive of experimental 1 is calculated according to $100mg/cm^2$ defect areas. The negative control group is covered by gauze and did not use any adhesive. According to the time interval of 0, 3, 7, 14, 21 days after operation, wound is photographed to observe the healing situation. The overall results are shown in Fig. 7.

As shown in Fig. 7a and Fig. 7b, the wound healing ratio is obviously increased while the wound is treated by medical adhesive. In the image of $0^{th}$ day, there is no significant difference that is found in the detect positions between two groups; after detect operation for 3 days, the wound healing ratio of treatment group (30.9±8.2%) is significantly higher than blank control group (10.4±1.5%); on the $7^{th}$ day, the blank control group shows wound healing rate of 24.4±5.5% and treatment group show wound healing rate of 54.5±12.4%; on $14^{th}$ day, treatment group shows the wound healing rate of 80.9±7.5% and blank control group shows wound healing rates of 58.2±11.4%; on day 21, the treatment group shows wound almost completely healed (98.1%±2.6%), and regenerated hair is covered the inner wound, while the blank control group shows wound healing rate of 71.9%±6.4%. For overall, the appearance of the wound in treatment group is significantly improved, while the non-operation of blank control group exhibits large and long scars.

In order to ensure the effect of operation of medical adhesive on epidermal regeneration and connective tissue contraction, H&E staining (as shown in Fig. 7c) is performed on day 21 after operation. The result of H&E staining shows that skin regeneration is thicker and ulcer area is smaller for SSAD treatment group. The statistical results also prove that the comparison results are statistically different (as shown in Fig. 7d). For Masson staining of SD rats wound sections (as shown in Fig. 7e), the results compared with normal skin, because of the proliferation and remodeling stage of granulation tissue at 21 days, the granulation tissue of SSAD treatment group is more mature and contained more blood vessels. Except for the epidermal healing, the SSAD treatment group formed a mature dermal structure (includes hair follicles and sebaceous glands), which is similar to normal tissue composition, but this phenomenon could not be observed in blank control group (as shown in Fig. 7f).

In order to determine the new shape of blood vessels at wound region, the angiogenesis-related CD31 and α-SMA is analyzed, the results as shown in Fig. 7g, in which DAPI staining is used to mark the position of nucleus in tissue and is provided for comparison. It can be seen from Fig. 7g, after operation $7^{th}$ day in SSAD treatment group, the content of positive cells of CD31 is 4.42±0.55% and the content of positive cells of blank control group is 1.48±0.39%. After operation $14^{th}$ day, the content of positive cells of CD31 of SSAD treatment group is 13.03±1.03% and the content of positive cells of CD31 of blank control group is 8.30±1.59%, the difference is statistically significant (p<0.001). The above results prove the SSAD treatment group can significantly increase the formation of new blood vessels at the injury site and promote the wound healing which is compared with the blank control group. It also proves that the medical adhesive of the present invention can promote tissue regeneration effect.

Experiment 6: Evaluation and effect of medical adhesive in vivo

Under deep general anesthesia with isoflurane, SD rats were in the prone position and an aseptic surgery is prepared for back of SD rats. A skin incision (3cm) outside the spinal axis is separated from the underlying subcutaneous tissue to provide enough space for implantation of medical adhesive. 100mg of giant salamander skin mucus dried powder is taken and is mixed with 200μl-600μl of PBS to form a medical adhesive. The, the medical adhesive is implanted into space of subcutaneous and is sutured the skin closely after implantation. The surrounding tissue and all-skin of wound are collected at 3, 7, and 14 days after operation to perform histological analysis to evaluate the degradation effect of medical adhesive. The degradation effect is shown in Fig. 8.

After medical adhesive is implanted in the body for 3, 7 and 14 days, H&E and Masson staining showed mild inflammation (as shown in Fig. 8a and Fig. 8b). After implantation for 3 days, the moderate acute inflammation reaction is observed in outmost layer of implanted medical adhesive and has typical inflammatory cells stained dark blue (the dark color cells in Fig. 8). After implantation for 7 days, the structure of medical adhesive began to lose it integrity, and is was filled with invading inflammatory cells, and no fibrous capsules are observed. That is, the response of host to the medical adhesive is weak. In addition, after implantation for 14 days, there is no medical adhesive remains on the implantation sites, so the skin structure is normal as the blank control group which indicates the medical adhesive can be degraded completely in the body.

By using lymphocytes (CD3) and macrophages (CD68) marks to stain, evaluate the cell characteristics of wound healing area, and the results are shown in Fig. 8c. i, v, and ix in Fig. 8c are partial enlarged image of the same field of view for 3 day after operation; similarly, ii, vi and x in Fig. 8c represents images of 21 days after operation. From Fig. 8c, it can be found that after 3 days of implantation, the lymphocyte infiltration rate around the graft in the medical adhesive treatment group is 0.23±0.06%, and there are few amounts of macrophage infiltration. The macrophage infiltration reached the maximum value on $7^{th}$ day (3.21±0.87%). With the passage of time, the number of lymphocytes and macrophages infiltrated decreased and disappeared thoroughly at $21^{st}$ day. At the same time, the above process is performed with quantitative statistical, the results as shown in Fig. 8d and Fig. 8e. there are statistical differences between each time point. This observation proves that giant salamander skin mucus hydrogel has good biocompatibility as a medical adhesive and is degraded thoroughly in the body and has no irritation, and there is no obvious immune rejection reaction.

By using liquid chromatography technology to detect the content of main substance of giant salamander skin mucus dried powder, the detection result finds that more than 87% of them are various amino acids.

Table 6 content of main substance of giant salamander skin mucus dried powder

| Sort | amino acid | content (mg/kg) | content percentage (%) |
|---|---|---|---|
| 1 | Lysine | 84718.11±705.005 | 8.47 |
| 2 | Threonine | 71375.08±714.28 | 7.14 |
| 3 | Glutamate | 69488.71±1151.98 | 6.95 |
| 4 | Glycine | 68308.8±594.745 | 6.83 |
| 5 | Aspartic acid | 62547.98±674.25 | 6.25 |
| 6 | Leucine | 61047.84±453.635 | 6.1 |
| 7 | Proline | 59855.79±493.31 | 5.99 |
| 8 | Isoleucine | 59898.02±417.785 | 5.99 |
| 9 | tyrosine | 55928.19±4420.91 | 5.59 |
| 10 | Valine | 53311.17±1022.665 | 5.33 |
| 11 | Serine | 52322.74±99.4 | 5.23 |
| 12 | phenylalanine | 50825.46±340.375 | 5.08 |
| 13 | alanine | 41874.33±328.675 | 4.19 |
| 14 | arginine | 38365.15±389.165 | 3.84 |
| 15 | histidine | 17974.12±178.975 | 1.8 |
| 16 | methionine | 14394.41±129.13 | 1.44 |
| 17 | Tryptophan | 9372.815±5.325 | 0.94 |
| 18 | cystine | 1680.24±71 | 0.17 |

Experiment 7: Evaluation and effect of different aqueous solution

The giant salamander skin mucus is obtained by electric stimulation, the dried powder is pulverized to sterilize with ethylene oxide. After standing for 48 hours to obtain the giant salamander skin mucus dried powder. The aqueous solution utilizes physiological saline (NaCl buffer), phosphate buffer (PBS), Tris buffer (TBS), citrate buffer, 2% chlorhexidine, blood, and platelet-rich plasma (PRP), platelet-rich plasma fibrin (PRF) or concentrated growth factor (CGF). The medical adhesive is prepared according to the method is described in experimental 1. The ratio parts by weight of giant salamander skin mucus dried powder and aqueous solution is shown in Table 7.

Table 7: ratio parts by weight of giant salamander skin dried powder and aqueous solution

| types of aqueous solution | parts (by weight) | types of aqueous solution | parts (weight) |
|---|---|---|---|
| physiological saline (NaCl buffer) | 1-3 | platelet-rich plasma (PRP) | 4-6- |
| phosphate buffer (PBS) | 1-3 | platelet-rich plasma fibrin (PRF) | 3-5 |
| Tris buffer (TBS) | 3-6 | Injectable platelet-rich plasma fibrin (iPRF) | 3-5 |
| citrate buffer | 2-5 | whole blood of human | 2-4- |
| 2% chlorhexidine | 2-4 | Whole blood and physiological saline (mixing ratio are 1:1) | 2-4- |
| Note: the amount of giant salamander skin mucus dried powder used in this table is 1 part by weight | | | |

The multiple cases of SD rats are treated with medical adhesive which is made of different aqueous solution according to different powder-to-water ratio. The treatment method is that the wound is covered by medical adhesive, in which the medical adhesive can cover and adhere the wound to exhibit the good adhesion ability. After 35 days, the rat heart, liver, spleen lung and kidney are taken for histological analysis to evaluate the biocompatibility of the medical adhesive. The blood sample is collected for blood biochemical analysis, which includes lactate dehydrogenase (LDH), blood urea nitrogen (BUN), alanine aminotransferase (ALT) and aspartate aminotransferase (AST) to evaluate the influence of medical adhesive for physiological value. During the treatment and subsequent observation (After injury for 35 days), there is no effect to observe on the overall health or behavior of the SD rats. In addition, H&E staining did not reveal any systemic damage to the histological examination of the heart, liver, spleen, ling, and kidney. Moreover, the toxic effects of kidney (blood urea nitrogen) and liver (aspartate aminotransferase, alanine aminotransferase) and general treatment group are evaluated. After topical administration of the medical gel, these parameters are within the normal reference value range compared with the blank control group, which indicates that there is no damage to the major organs after treatment of medical gel.

Experiment 8: Evaluation and effect of biocompatibility of medical adhesive in vivo

The present invention utilizes a plurality of aqueous solution and medical adhesive with different powder-to-water ratios to treat multiple case of SD rats. The treatment method is that the appropriate medical adhesive is used to suture the subcutaneous of SD rats. After operation for 35 days, the rat heart, liver, spleen, lung, and kidney are taken for histological analysis to evaluate the biocompatibility of the medical adhesive. The blood samples are collected for blood biochemical analysis, which includes lactate dehydrogenase (LDH), blood urea nitrogen (BUN), alanine aminotransferase (ALT) and aspartate aminotransferase (AST) to evaluate the influence of medical adhesive for physiological value. The medical adhesive can adhere to the skin incision to promote wound healing in diabetic models. However, the assessment of long-term toxicity in vivo is critical for biological applications. During the treatment and subsequent observation (after injury for 35 days), there is no effect to observe on the overall health or behavior of the SD rats. In addition, H&E staining did not reveal any systemic damage to the histological examination of the heart, liver, spleen, ling, and kidney. Moreover, the toxic effects of kidney (blood urea nitrogen) and liver (aspartate aminotransferase, alanine aminotransferase) and general treatment group are evaluated. After topical administration of the medical gel, these parameters are within the normal reference value range compared with the blank control group, which indicates that there is no damage to the major organs after treatment of medical gel.

Experiment 9: Evaluation of the therapeutic effect of medical adhesive on tendons

The SD rats are used as experimental animals, and normal group use normal rats without any operation; SSAD treatment group is treated with the medical adhesive of the present invention after the Achilles tendon of SD rats are cut; the blank control group is treated with PBS (phosphate buffered saline solution) after the Achilles tendon of SD rats have been cut, thereby let the Achilles tendon of SD rats heal naturally. The specific method is to select 20 3-month-old SPF order male SD rats (which is provided by the Experimental Animal Center of Chongqing Medical University) and weight of each SD rats is 280±30g. Adaptive feeding for 7 days, the operation of tendon dissection is performed according to the established rats Achilles tendon defect model (as shown in Fig.9). One hind leg of SD rats is selected to use as the experimental group (for example right hind leg) and the skin of SD rats is cut to separate the aponeurosis (as shown in Fig. 9a). The Achilles tendon and Plantaris tendon (as shown in Fig. 9b) are exposed. The Achilles tendon of SD rats is cut off to prevent internal fixation, and the Plantaris tendon is also cut off (As shown in Fig. 9c). The aponeurosis can enhance the growth factor to promote the healing ability of Achilles tendon. It should be noted that the over-injure of the tendon is to be prevented when Achilles tendon is cut and sutured.
The SD rats are randomly divided into SSAD treatment group and blank control group, each with 10 SD rats, and the Achilles tendon of each 10 SD rats are cut according to above surgical method. The SSAD treatment group is treated with the medical adhesive obtained in experimental 1: 10mg of giant salamander skin mucus dried powder is taken to apply on the stump of Achilles tendon (as shown in Fig. 9d), and is mixed with 30μL of PBS to form a hydrogel wraparound the stump of Achilles tendon of SD rats (as shown in Fig. 9e) to seal the wound (as shown in Fig. 9f). The blank control group instills 30μL of PBS into stump of Achilles tendon of SD rats to seal the wound.
After the operation, all animals are free to take water and food (standard feed), and are fed in a clean environment with a room temperature of 25°C-28°C and a humidity of 75%-80%. After operation for 28th day, the action of each SD rats is to analyzed by using small animal gait instrument, some samples are taken for mechanical testing (n=6), and the rest of Achilles tendon of SD rats are fixed, and then the paraffin section HE and Masson staining are performed.
Compare the tendon strength of SSAD treatment group and blank control group, the mechanical testing method is carried out according to the following method. First, the complete tendon tissue is separated and use two scalpel blades to cut away the surrounding original muscle tissue at a safe distance of 5 mm above and below the tendon tissue while retaining all the tendon tissue. Both two ends of 5mm of muscle are fixed on universal testing machine (MTS Criterion, Model 43, USA). The parameter used in mechanical testing is 15mm/min. When the maximum force that new Achilles tendon tissue can bear is reached, the tendon is broken, the universal testing machine records the curve of force and displacement. The maximum broken intensity (the unit represented as N/m$^2$) is calculated according to the maximum tensile force and the cross-sectional area of the new Achilles tendon tissue measured in advance, and Young's modulus can be calculated according to the slope of curve.
The mechanical testing showed that the maximum load tension of the tendon in SSAD treatment group (25.6N±8.2N) is significantly higher than that in blank control group (13.8±3.9N) (P < 0.05) after treatment for 21 days. At the same time, cross-sectional area (10.5±4.7mm$^2$) of tendon in SSAD treatment group is smaller than that in blank control group (16.1±5.8mm$^2$). The tenon intensity in SSAD treatment group (2.8±1.1MPa) is higher than that in blank control group (0.9±0.2MPa) (P<0.05). The stiffness of tendon in SSAD treatment group (17.7±7.5N/mm) is higher than that in blank control group (6.9±1.2N/mm) (P<0.05). The elastic modulus of SSAD treatment group (14.6±6.9MPa) is higher than that of blank control group (3.0±0.6MPa).

After treatment for 28 days, the maximum load tension (52.8N±9.8N) of the tendon in SSAD treatment group is higher than that in blank control group (33.3±8.3N) (P<0.05). At the same time, the cross-sectional area (7.7±1.1mm$^2$) of tendon in SSAD treatment group is smaller than that in blank control group (9.3±1.6mm$^2$). The tendon intensity (7.1±1.9MPa) of SSAD treatment group is higher than that of blank control group (3.6±0.9MPa) (P<0.05). The stiffness of tendon of SSAD treatment group is higher than that of blank control group (15.3±3.0N/mm) (P<0.05). The elastic modulus (36.3±11.2MPa) SSAD treatment group is higher than that of blank control group (15.0±3.5MPa) (P<0.05).

The maximum lad tension of normal tendon is 42.3±2.9N, the intensity is 14.0±5.8MPa, the stiffness is 29.8±7.3N/mm and the elastic modulus is 44.4±12.9MPa.

The maximum load tension of tendon in SSAD treatment group is even high than that of normal group, but the area of SSAD treatment group is larger than that of normal group (7.7±1.1mm$^2$ vs. 3.5±1.2mm$^2$). The tendon intensity (14.0±5.8MPa) of normal group is still much higher than that of SSAD treatment group (7.1±1.9MPa), which indicates that the tendon quality of normal group is higher than that of SSAD treatment group. The reason why the tendon load of SSAD treatment group is even exceeded that of the tendon of normal group should be due to early tendon healing accompanied by hyperplasia. It shows that the medical adhesive of the present invention can improve the strength of the healed Achilles tendon and reduce the risk of re-broken of tendon.

Compare the running state of SSAD treatment group, blank control group and normal group, the animal models of SSAD treatment group and blank control group show normal left hind legs and surgery on the right hind legs. The left and right legs of normal group are not to perform operating. A rodent gait instrument (Catwalk XT gait analysis system, Nordas, the Netherlands) is used to evaluate the action ability of the SD rats after the operation, and the software built-in machine is used to export the detection data, and statistical analysis is performed by SPSS statistics 25 software (IBM, Armonk, NY, USA). The data are expressed as average ± standard deviation. Two independent samples t-tests to calculate whether there is statistical difference.

The footprint area, maximum contact area, the duty ratio, the proportion of the maximum intensity, the maximum intensity of the maximum contact area, average intensity of maximum contact area, maximum intensity, average intensity, and the average intensity of top 15 largest sole pressures are measured respectively. On 21 days, 10 rats are measured in the blank control group and 10 rats are measured in SSAD treatment group. On 28 days, 4 rats are measured in blank control group, 5 rats are measured in SSAD treatment group and gait data of 4 normal rats are measured at the same time. The data are expressed as mean ± standard deviation. According to existing literature reports and comparison with the rats in normal group, the healing performance is good of Achilles tendon is that except for the increase in the proportion of reaching maximum intensity, the other index is all smaller.

After the operation for 21 days, the footprint area (1.68±0.55 cm$^2$) of rats in SSAD treatment group is smaller than that of blank control group (2.15±0.33 cm$^2$) (P<0.05). The duty ratio (the time for foot touching the ground/walking period) (71.7±3.8%) of rats in SSAD treatment group is lower than that in blank control group (77.1±4.8%) (P<0.05) (as shown in Fig. 10). The maximum intensity (176.3±32.3) of maximum contact area of SSAD treatment group, average intensity of maximum contact area (90.6±13.1), maximum intensity (186.0±25.0), average intensity (96.8±13.9) and average intensity of top 15 largest sole pressures (168.8±32.4) in SSAD treatment group are smaller than the maximum intensity of maximum contact area (199.3±9.7%), average intensity (103.0±6.5) of maximum contact area, maximum intensity (205.2±8.1) (as shown in Fig. 11), average intensity (109.7±6.9), average intensity (196.2±13.1) of top 15 largest sole pressures of blank control group. In the SSAD treatment group, the proportion of the time to touch the maximum intensity of the sole of the foot (63.8±12.7%) is greater than that in the blank control group (47.7±13.9%). The weight of the rats in the SSAD treatment group and the blank control group is similar, thereby the interference of body weight on foot strength is eliminated.

After the operation for 28 day, the indicators of statistical difference between SSAD treatment group and blank control group includes maximum contact area (0.8±0.1cm$^2$ vs. 1.3±0.3cm$^2$) (as shown in Fig. 12), maximum intensity (154.9±21.0 vs. 192±10.8), average intensity of maximum contact area (67.5±2.7 vs. 80.0±8.4), footprint area (1.2±0.2cm$^2$ vs. 1.8±0.4cm$^2$), maximum intensity (173.3±16.8 vs. 200.2±7.0) (as shown in Fig .11), top 15 average intensities (146.2±13.6 vs. 182.1±17.8) (P<0.05) of maximum sole pressure. The data of normal group is the footprint area (0.45cm$^2$±0.14cm$^2$), maximum contact area (0.32cm$^2$±0.11cm$^2$), the duty ratio (the time for foot touching the ground/walking period) (62.1±2.9%), the time duty ratio for sole touches the ground to reach the maximum intensity (70.7±12.8%), maximum intensity of maximum contact area (83.4±20.2), average intensity of maximum contact area (47.1±7.4), maximum intensity (93.1±21.3), average intensity(51.8±7.1), and top 15 average intensity (74.1±16.2) of maximum sole pressure. Regardless of whether it is 21 days or 28 days, the data of the SSAD treatment group is closer to the normal group according to above data, which indicates that medical adhesive provided by the present invention can improve the action ability of injured Achilles tendon, and the walking duty ratio of the right hind leg of rats at 21 days (After operation for 21 day) is shown in Fig. 10, which shows the walking duty period of right hind leg (foot touches time/walking period). The walking period of three groups of SD rats is elongated to same periods, and the time sequence from longest to shortest of foot touches time is blank control group, SSAD treatment group, normal group, that is, the sequence of duty ratio is blank control group, SSAD treatment group, and normal group. The maximum press intensity of the footprint of right leg of rats on 21st and 28th days is shown in Fig. 11, which indicates the three-dimensional schematic of showing footprint press intensity of right leg of rats. X axis represents the length of footprint, Y axis represents the width of footprint, and Z axis represents the maximum pressure intensity of footprint. The results show that the sequence of maximum pressure intensity is blank control group, SSAD treatment group and normal group. The maximum area of footprint of right leg of rats on 21 days and 28 days are shown in Fig. 12, which indicates the sequence of footprint area of right leg of rats on 21 day and 28 day is blank control group, SSAD treatment group and normal group. The data of all gait indicators are shown in Table 8.

Table 8 Results of gait instrument

| index | 21day blank control group | 21day treatment group | SSAD P | 28 days blank control group | 28 days treatment group | SSAD P |
|---|---|---|---|---|---|---|
| footprint area | 2.1±0.33 | 1.7±0.55 | 0.036 | 1.8±0.4 | 1.2±0.2 | 0.026 |
| maximum contact area | 1.6±0.2 | 1.3±0.4 | 0.113 | 1.3±0.3 | 0.8±0.2 | 0.018 |
| duty ratio | 77.1±4.8 | 71.7±3.8 | 0.016 | 68.5±5.1 | 67.2±5.2 | 0.771 |
| time duty of maximum intensity | 47.7±13.9 | 63.8±12.7 | 0.016 | 49.7±19.2 | 68.4±13.6 | 0.190 |
| maximum intensity of maximum contact area | 199.3±9.7 | 176.3±32.3 | 0.065 | 192±10.7 | 154.9±21.0 | 0.034 |
| average intensities of maximum contact area | 103.0±6.5 | 90.6±13.1 | 0.016 | 80.0±8.4 | 67.5±2.7 | 0.030 |
| maximum intensity | 205.2±8.1 | 186.0±25.0 | 0.050 | 200.3±7.0 | 173.3±16.8 | 0.041 |
| average intensity | 109.7±6.9 | 96.8±13.9 | 0.026 | 86.8±10.8 | 72.7±4.2 | 0.053 |
| top 15 average intensities of maximum sole pressure intensity | 196.2±13.2 | 168.8±32.4 | 0.036 | 182.1±17.8 | 146.2±13.6 | 0.023 |

After the operation for 28 day, HE sections of the right hind leg of two rats in experiment group and blank control group respectively are taken for staining. It is found that the blank control group includes fewer collagen fiber, no orientation, and the fat cells infiltration can be found obviously. The collagen fibers of SSAD treatment group (as shown in Fig. 13) is rich, the fiber showed a certain orientation, and the fat cell infiltration cannot be seen obviously. It can be showed that the treatment effect is obviously to the Achille tendon. The above results show that the Achilles tendon healing in SSAD treatment group is better than that in the blank control group. However, due to the recovery time (28 days) is short, the action ability of rats in SSAD treatment control group has not yet recovered to a normal state.

From above descriptions, the present invention provides a medical adhesive prepared by unmodified giant salamander skin mucus and its use. It can be applied to tissue adhesion on wound through varieties usage methods to promote the wound healing, and the comprehensive performance is better than the current medical adhesive.

During the use of medical adhesive, the gel can quickly (time is less than 60 seconds) seal the opening of wound of bleeding on the back of the rat, so the full-thickness skin defect of diabetic SD rats can be treated effectively. In addition, the medical adhesive can be thoroughly degraded in the body within 2 weeks and medical adhesive has low inflammatory foreign substance reaction. Therefore, the medical adhesive of the present invention has the advantages of convenient operation, easy modification, good biocompatibility, as well as the comprehensive effects of promoting tissue regeneration, promotion of wound healing, anti-oxidation and antibacterial. It provides a practical seamless selection for skin, dense connective tissue, fragile organs and inaccessible internal tissue wound. In addition, the low cost of giant salamander skin mucus is to be considered and the environmentally friendly processing steps, it is expected that the medical adhesive proposed in the present invention can be used as an elastic and malleable medical adhesive product to overcome the defects and limitations of the current products, and is widely used.

The above descriptions are only preferred embodiments of the present invention and are not intended to limit the scope of rights of the present invention. At the same time, the above descriptions should be understood and implemented by those skilled in the relevant technical fields, so the others do not deviate from what is disclosed in the present invention. All equivalent changes or modifications made in the spirit should be included in the scope of the patent application.

**Claims**

1. A medical adhesive, comprising:

   a giant salamander skin mucus dried powder; and
   an aqueous solution, wherein the ratio parts by weight of the giant salamander skin dried powder and the aqueous solution is 1:1 to 1:6.

2. The medical adhesive according to claim 1, wherein the medical adhesive is in gel form.

3. The medical adhesive according to claim 1, wherein the weight percentage of the giant salamander skin mucus dried powder in the medical adhesive ranges from 14.2% to 50%.

4. The medical adhesive according to claim 1, wherein the aqueous solution is selected from the group consisting of distilled water, physiological buffer solution, chlorhexidine, blood, plasma, blood cell preparation, platelet-rich plasma, platelet-rich plasma fibrin or any combination of the above.

5. The medical adhesive according to claim 4, wherein the physiological buffer solution is selected from the group consisting of normal saline, phosphate buffer, Tris buffer, citate buffer or any combination of the above.

6. The medical adhesive according to claim 2, wherein the medical adhesive has a porous structure.

7. The medical adhesive according to claim 6, wherein the average diameter of hole of the porous structure is smaller than 116μm.

8. The medical adhesive according to claim 1, wherein the average diameter of hole of the porous structure is between 6μm and 37μm.

9. The medical adhesive according to claim 1, wherein the size of the giant salamander skin mucus dried powder is -20 mesh.

10. The medical adhesive according to claim 1, wherein the size of the giant salamander skin mucus dried powder is between -60 mesh and +300 mesh.

11. A use of the medical adhesive according to any one of claims 1-10, the medical adhesive is provided for wound adhesin, repair and healing.

12. The use of the medical adhesive according to claim 11, wherein the wound includes wounds located in the epidermis, dermis and subcutaneous connective tissue.

13. The use of the medical adhesive according to claim 11, wherein the wound includes wounds located in skeletal muscles, tendons, ligaments, bones and/or connective tissue around bones.

14. The use of the medical adhesive according to claim 11, wherein the giant salamander skin mucus dried powder and the aqueous solution are directly provided for applying on the wound to formed as the medical adhesive on the wound.

15. The use of the medical adhesive according to claim 11, wherein a giant salamander skin mucus dried powder and an aqueous solution are preliminarily formed as a medical adhesive and the medical adhesive is then used to apply on the wound or to seal the wound.

16. A preparation method of medical adhesive according to any one of claims 1-10, comprising:

providing a giant salamander skin mucus dried powder;

sterilizing the giant salamander skin mucus dried powder;

mixing a sterilized giant salamander skin mucus dried powder and an aqueous solution to carry out a gelation process to form the medical adhesive in a gel form, wherein the ratio parts by weight of the giant salamander skin freeze-dried powder and the aqueous solution is 1:1 to 1:6, the weight percentage of the giant salamander skin mucus freeze-dried powder in the medical adhesive ranges from 14.2% to 50%.

17. The preparation method of medical adhesive according to claim 16, wherein the step of providing the giant salamander skin mucus dried powder further comprising:

obtaining the giant salamander skin mucus from a living giant salamander skin;

freezing and drying the giant salamander skin mucus; and

grinding and crushing the frozen and dried giant salamander skin mucus to form the giant salamander skin mucus dried powder.

18. The preparation method of medical adhesive according to claim 16 or 17, wherein the size of the giant salamander skin mucus dried powder is -20 mesh.

19. The preparation method of medical adhesive according to claim 16 or 17, wherein the size of the giant salamander skin mucus dried powder is between -60 mesh and +300 mesh.

20. The preparation method of medical adhesive according to claim 16, wherein the step of sterilization is achieved by using ethylene oxide.

21. The preparation method according to claim 16, wherein the aqueous solution is selected from the group consisting of distilled water, physiological buffer, chlorhexidine, blood, plasma, blood cell preparation, platelet-rich plasma, platelet-rich plasma fibrin or any combination of the above.

22. The preparation method of medical adhesive according to claim 21, wherein the physiological buffer solution is selected from the group consisting of normal saline, phosphate buffer, Tris buffer, citrate buffer or any combination of the above.

The mucus is obtained from the giant salamander skin, and the giant salamander skin mucus is frozen and dried ⟶ 1

Freeze-dried giant salamander skin mucus is crushed under frozen state, and then sieve to obtain a powder with specific particle range ⟶ 2

the powder is sterilized to obtain a giant salamander skin mucus freeze-dried powder ⟶ 3

The sterilized giant salamander skin mucus dried powder is mixed with the aqueous solution according to the ratio parts by weight of 1:1 to 1:6 to form a gel-form medical adhesive ⟶ 4

Fig. 1

Fig. 2

Fig. 3A

Fig. 3B

Fig. 3C

Fig. 4A

Fig. 4B

Fig. 4C

Fig. 5A

Fig. 5B

Fig. 5C

Fig. 6

Fig. 7

Fig. 8

Fig. 9

Fig. 10

day 21 day 28

Empty control group

SSAD group

normal group

Fig. 11

day 21 day 28

Empty control group

SSAD group

normal group

Fig. 12

a

b

Fig. 13

## INTERNATIONAL SEARCH REPORT

| International application No. |
| --- |
| **PCT/CN2020/076763** |

**A.    CLASSIFICATION OF SUBJECT MATTER**

A61L 24/00(2006.01)i；  A61K 35/65(2015.01)i；  A61K 9/06(2006.01)i；  A61P 17/02(2006.01)i

According to International Patent Classification (IPC) or to both national classification and IPC

**B.    FIELDS SEARCHED**

Minimum documentation searched (classification system followed by classification symbols)

A61L,A61K,A61P

Documentation searched other than minimum documentation to the extent that such documents are included in the fields searched

Electronic data base consulted during the international search (name of data base and, where practicable, search terms used)

CNKI, CNABS, VEN, CNTXT, USTXT, EPTXT, WOTXT: 粘合, 黏合, 鲵, 娃娃鱼, 蝾螈, 干粉, 水性, 溶液, 凝胶, 蒸馏水, 生理缓冲液, 生理盐水, 氯己定, 血液, 血浆, 血细胞制剂, 富血小板血浆, 富血小板血浆纤维蛋白, 磷酸盐缓冲液, Tris缓冲液, 柠檬酸盐缓冲液, 创面, 创伤, 修复, 愈合, 灭菌, 混合, 冷冻干燥, 研磨, 粉碎, adhesi+, bond+, salamander?, baby fish??, andrias davidianus, dry powder, aqueous, solution, gel, distilled water, physiological buffer, physiological saline, chlorhexidine, blood, plasma, blood cell preparation, platelet-rich plasma, platelet-rich plasma fibrin, phosphate buffer, Tris buffer, citrate buffer, wound surface, wound, repair+, heal+, steriliz+, mix+, fr??z?? drying, grind+, crush+

**C.    DOCUMENTS CONSIDERED TO BE RELEVANT**

| Category* | Citation of document, with indication, where appropriate, of the relevant passages | Relevant to claim No. |
| --- | --- | --- |
| PX | CN 110559469 A (STOMATOLOGICAL HOSPITAL OF CHONGQING MEDICAL UNIVERSITY) 13 December 2019 (2019-12-13) <br> description, paragraphs [0007]-[0043] | 1-22 |
| X | CN 107236134 A (WEI, Hong et al.) 10 October 2017 (2017-10-10) <br> description, paragraphs [0006]-[0023] and [0086]-[0087] | 1-22 |
| X | CN 104815349 A (CHONGQING KUIXU BIOLOGICAL SCIENCE AND TECHNOLOGY CO., LTD. et al.) 05 August 2015 (2015-08-05) <br> description, paragraphs [0005]-[0032] | 1-22 |
| X | CN 104740678 A (CHONGQING KUIXU BIOLOGICAL SCIENCE AND TECHNOLOGY CO., LTD. et al.) 01 July 2015 (2015-07-01) <br> description, paragraphs [0008]-[0036] | 1-22 |
| X | CN 106581736 A (ANSHUN SIHAI AGRICULTURAL SCIENCE AND TECHNOLOGY DEVELOPMENT CO., LTD.) 26 April 2017 (2017-04-26) <br> description, paragraphs [0007]-[0017] | 1-22 |

☑ Further documents are listed in the continuation of Box C.　　☑ See patent family annex.

\*    Special categories of cited documents:

"A"    document defining the general state of the art which is not considered to be of particular relevance

"E"    earlier application or patent but published on or after the international filing date

"L"    document which may throw doubts on priority claim(s) or which is cited to establish the publication date of another citation or other special reason (as specified)

"O"    document referring to an oral disclosure, use, exhibition or other means

"P"    document published prior to the international filing date but later than the priority date claimed

"T"    later document published after the international filing date or priority date and not in conflict with the application but cited to understand the principle or theory underlying the invention

"X"    document of particular relevance; the claimed invention cannot be considered novel or cannot be considered to involve an inventive step when the document is taken alone

"Y"    document of particular relevance; the claimed invention cannot be considered to involve an inventive step when the document is combined with one or more other such documents, such combination being obvious to a person skilled in the art

"&"    document member of the same patent family

| Date of the actual completion of the international search | Date of mailing of the international search report |
| --- | --- |
| **13 May 2020** | **22 May 2020** |

| Name and mailing address of the ISA/CN | Authorized officer |
| --- | --- |
| **China National Intellectual Property Administration** <br> **No. 6, Xitucheng Road, Jimenqiao Haidian District, Beijing 100088** <br> **China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2020/076763** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 104758978 A (CHONGQING KUIXU BIOLOGICAL SCIENCE AND TECHNOLOGY CO., LTD. et al.) 08 July 2015 (2015-07-08)<br>entire document | 1-22 |
| A | CN 108421080 A (WEI, Hong et al.) 21 August 2018 (2018-08-21)<br>entire document | 1-22 |
| A | WO 0222756 A1 (LUMINIS PTY LTD. et al.) 21 March 2002 (2002-03-21)<br>entire document | 1-22 |

Form PCT/ISA/210 (second sheet) (January 2015)

**INTERNATIONAL SEARCH REPORT**

International application No.

**PCT/CN2020/076763**

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **11-15**
because they relate to subject matter not required to be searched by this Authority, namely:

[1]   Claims 11-15 relate to uses of a medical adhesive, which fall within a method for treating a living human or animal body, and fall within the subject matter set out in PCT Rule 39.1, for which a search by the International Searching Authority is not required. "Said medical adhesive being applied to adhesion, plerosis and healing of a wound" in claim 11 is reasonably expected to be "said medical adhesive being used in preparation of a material for adhesion, plerosis and healing of a wound". The search is carried out with respect to the subject matter after being amended as reasonably expected.

2. ☐  Claims Nos.:
because they relate to parts of the international application that do not comply with the prescribed requirements to such an extent that no meaningful international search can be carried out, specifically:

3. ☐  Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.

**PCT/CN2020/076763**

| Patent document cited in search report | | | Publication date (day/month/year) | Patent family member(s) | | | Publication date (day/month/year) |
|---|---|---|---|---|---|---|---|
| CN | 110559469 | A | 13 December 2019 | None | | | |
| CN | 107236134 | A | 10 October 2017 | CN | 107236134 | B | 17 January 2020 |
| CN | 104815349 | A | 05 August 2015 | CN | 104815349 | B | 24 October 2017 |
| CN | 104740678 | A | 01 July 2015 | CN | 104740678 | B | 24 October 2017 |
| CN | 106581736 | A | 26 April 2017 | None | | | |
| CN | 104758978 | A | 08 July 2015 | CN | 104758978 | B | 16 June 2017 |
| CN | 108421080 | A | 21 August 2018 | None | | | |
| WO | 0222756 | A1 | 21 March 2002 | AU | PR024300 | D0 | 12 October 2000 |
| | | | | AU | PR024300 | A0 | 12 October 2000 |

Form PCT/ISA/210 (patent family annex) (January 2015)

**EP 3 932 436 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 104815349 B
- CN 106581736 A
- CN 108421080 A

**Non-patent literature cited in the description**

- *Biomaterials science,* 2018, vol. 6, 2757-72